(19) Europäisches Patentamt
European Patent Office
Office européen des brevets

(11) Veröffentlichungsnummer : **0 421 221 B1**

(12) # EUROPÄISCHE PATENTSCHRIFT

(45) Veröffentlichungstag der Patentschrift :
**30.11.94 Patentblatt 94/48**

(51) Int. Cl.$^5$ : **G03C 7/305,** G03C 7/36

(21) Anmeldenummer : **90118264.2**

(22) Anmeldetag : **22.09.90**

(54) **Farbfotografisches Aufzeichnungsmaterial mit einem DIR-Kuppler.**

(30) Priorität : **05.10.89 DE 3933238**

(43) Veröffentlichungstag der Anmeldung :
**10.04.91 Patentblatt 91/15**

(45) Bekanntmachung des Hinweises auf die Patenterteilung :
**30.11.94 Patentblatt 94/48**

(84) Benannte Vertragsstaaten :
**BE DE FR GB**

(56) Entgegenhaltungen :
**EP-A- 0 127 063**

(73) Patentinhaber : **Agfa-Gevaert AG**
**Kaiser-Wilhelm-Allee**
**D-51373 Leverkusen (DE)**

(72) Erfinder : **Odenwälder, Heinrich, Dr.**
**Am Arenzberg 37**
**D-5090 Leverkusen 3 (DE)**
Erfinder : **Krüger, Thomas, Dr.**
**Friedrich-Engels-Strasse 18**
**D-5090 Leverkusen (DE)**
Erfinder : **Schumann, Hans-Joachim, Dr.**
**Leopold-Gmelin-Strasse 7**
**D-5000 Köln 80 (DE)**

EP 0 421 221 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein farbfotografisches Aufzeichnungsmaterial mit mindestens einer lichtempfindlichen Silberhalogenidemulsionsschicht, die einen Kuppler enthält, der bei Farbentwicklung einen Entwicklungsinhibitor freisetzt.

Es ist bekannt, die chromogene Entwicklung in Gegenwart von Verbindungen durchzuführen, die bei der Entwicklung bildmäßig diffusionsfähige Substanzen freisetzen, die eine bestimmte Wirkung entfalten, beispielsweise die Entwicklung von Silberhalogenid zu beeinflussen vermögen. Falls diese Wirkung darin besteht, daß die weitere Entwicklung inhibiert wird, werden derartige Verbindungen als sogenannte DIR-Verbindungen (DIR = development inhibitor releasing) bezeichnet. Bei den DIR-Verbindungen kann es sich um solche handeln, die unter Abspaltung eines Inhibitorrestes mit dem Oxidationsprodukt eines Farbentwicklers zu einem Farbstoff reagieren (DIR-Kuppler), oder um solche, die den Inhibitor freisetzen ohne gleichzeitig einen Farbstoff zu bilden. Letztere werden auch als DIR-Verbindungen im engeren Sinne bezeichnet.

DIR-Kuppler sind beispielsweise bekannt aus US-A-3 148 062, US-A-3 227 554, US-A-3 615 506, US-A-3 617 291 und DE-A-24 14 006.

Bei freigesetzten Entwicklungsinhibitoren handelt es sich in der Regel um heterocyclische Mercaptoverbindungen oder um Derivate des Benzotriazols. DIR-Kuppler, die als Entwicklungsinhibitor monocyclische Triazole freisetzen, sind beispielsweise beschrieben in DE-A-28 42 063 und EP-A-0 272 573. Durch Anwendung von DIR-Verbindungen kann eine Vielzahl von fotografischen, die Bildqualität beeinflussenden Effekten bewirkt werden. Solche Effekte sind beispielsweise die Erniedrigung der Gradation, die Erzielung eines feineren Farbkorns, die Verbesserung der Schärfe durch den sogenannten Kanteneffekt und die Verbesserung der Farbreinheit und der Farbbrillanz durch sogenannte Interimageeffekte. Zu verweisen ist beispielsweise auf die Publikation "Development-Inhibitor-Releasing (DIR) Couplers in Color Photography" von C.R. Barr, J.R. Thirtle und P.W. Vittum, Photographie Science and Engineering 13, 74 (1969).

Die farblos kuppelnden DIR-Verbindungen haben vor den farbig kuppelnden DIR-Kupplern den Vorteil, daß sie universell einsetzbar sind, so daß die gleiche Verbindung ohne Rücksicht auf die zu erzeugende Farbe in allen lichtempfindlichen Schichten eines farbfotografischen Aufzeichnungsmaterials verwendet werden kann. DIR-Kuppler können dagegen wegen der auf ihnen erzeugten Farbe meist nur in einem Teil der lichtempfindlichen Schichten verwendet werden, falls nicht die auf sie zurückzuführende Farbnebendichte in den anderen Schichten tolerierbar ist. Diesem Vorteil der DIR-Verbindungen steht als Nachteil gegenüber, daß sie im allgemeinen weniger reaktiv sind als die DIR-Kuppler. In der Praxis hat man sich daher meist darauf beschränkt, DIR-Kuppler zu verwenden und zwar notfalls zwei oder mehrere verschiedene im gleichen Aufzeichnungsmaterial, wobei den unterschiedlich spektral sensibilisierten Schichten verschiedene DIR-Kuppler nach Maßgabe der aus den letzteren erzeugten Farbe zugeordnet werden können.

Es ist normalerweise wichtig, daß der Entwicklungsinhibitor bei Entwicklung rasch aus dem Kuppler freigesetzt wird, weil er den weiteren Verlauf der Entwicklung beeinflussen soll. Es ist daher sehr erwünscht, wenn die betreffenden Kuppler sehr aktiv sind. Hochaktive DIR-Kuppler sind beispielsweise beschrieben in EP-A-0 287 833. DIR-Kuppler des Standes der Technik tendieren unter forcierten Lagerbedingungen, die für fotografische Materialien, insbesondere Filme, durchaus noch realistisch sind, oft dazu ihre Aktivitäten zu verlieren oder den Inhibitor in unkontrollierter Weise freizusetzen. Dies führt zu höchst unerwünschten Effekten wie Verminderung der Empfindlichkeit und Farbreproduzierbarkeit. Es ist daher weiter erwünscht, daß DIR-Kuppler sich bei Lagerung innerhalb eines weiten Bereiches von äußeren Bedingungen (Temperatur, Feuchtigkeit) äußerst stabil verhalten.

Der Erfindung liegt die Aufgabe zugrunde, ein farbfotografisches Aufzeichnungsmaterial anzugeben, das DIR-Kuppler mit einem an die Kupplungsstelle gebundenen Silberhalogenidentwicklungsinhibitor enthält, aus denen bei der Entwicklung der Inhibitor rasch freigesetzt wird.

Gegenstand der Erfindung ist ein farbfotografisches Aufzeichnungsmaterial mit mindestens einer lichtempfindlichen Silberhalogenidemulsionsschicht und einem dieser zugeordneten DIR-Kuppler, dadurch gekennzeichnet, daß der Kuppler der folgenden Formel I entspricht

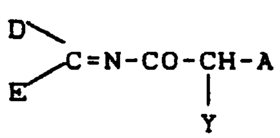

(I)

worin bedeuten

A Halogen, -OR, -SR, -CN, -COR, -CONHR, COOR, $-SO_2R$, $-SO_2NHR$,

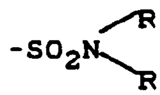, 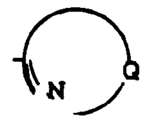, 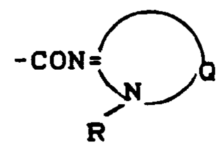

oder 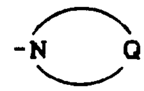 ;

R Alkyl, Aralkyl, Aryl oder Hetaryl;

Q einen Rest zur Vervollständigung eines 5- oder 6-gliedrigen heterocyclischen Ringes;

D,E Reste, die gleich oder verschieden sind und an das C-Atom der C=N-Gruppe über ein C-, O-, S- oder N-Atom gebunden sind, einschließlich solcher Reste, die gemeinsam zusammen mit dem C-Atom der C=N-Gruppe einen carbocyclischen oder heterocyclischen Ring vervollständigen;

Y einen bei Farbentwicklung freisetzbaren Rest, der gegebenenfalls nach Abspaltung eines Teils desselben eine fotografische Wirksamkeit entfaltet, insbesondere einen Rest mit Silberhalogenidentwicklungsinhibierungsfunktion.

Ein in Formel I bei A erwähnter Rest R kann Alkyl, Aralkyl oder Aryl sein. Hierbei ist Alkyl geradkettig oder verzweigt und enthält bis zu 18 C-Atome; Aralkyl ist beispielsweise Benzyl und Aryl ist beispielsweise Phenyl. Die Alkyl-, Aralkyl- und Arylreste können gegebenenfalls weiter substituiert sein, z.B. durch Halogen, $-CF_3$, -CN, Alkyl, Alkoxy, Acylamino, Carbamoyl, Alkoxycarbonyl, Sulfamoyl. Zwei Reste R können gemeinsam zusammen mit einem N-Atom, an das sie gebunden sind, eine cyclische Aminogruppe, z.B. Pyrrolidino, Piperidino, Morpholino, vervollständigen.

Ein durch Q vervollständigter heterocyclischer Ring ist beispielsweise

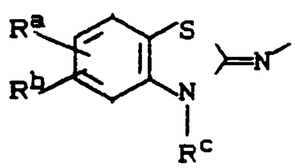 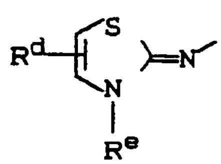

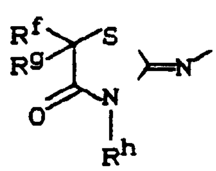 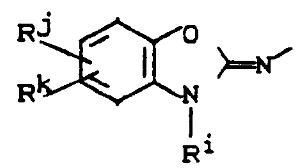

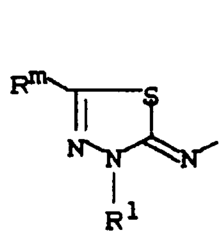 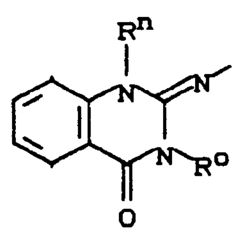

Die Reste $R^a$ bis $R^q$ sind Reste wie sie bei üblichen Synthesen dieser heterocyclischen Ringe vertreten sein können, z.B. Alkyl, Aryl, Hetaryl, Halogen, Alkoxy, Acylreste, Nitro und Sulfamoyl.

Die Reste $R^c$, $R^e$, $R^h$, $R^i$, $R^l$, $R^n$ und $R^o$ sind in der Regel Reste wie z.B. Alkyl, Aryl.

Die durch D und E dargestellten Reste sind insbesondere über ein S-Atom an das C-Atom der C=N-Gruppe gebundene Reste, z.B. Alkylthio, Aralkylthio oder Arylthio. Heterocyclische Ringe, die durch D und E vervollständigt werden, sind beispielsweise die Ringe von Benzoxozol, Benzthiazol, Chinazolon, Thiazolidon.

Der in Formel I durch Y dargestellte Rest mit fotografischer Wirksamkeit ist insbesondere ein Rest mit Silberhalogenidentwicklungsinhibierungsfunktion und entspricht der Formel

$$-(TIME)_n-INH .$$

Hierin ist INH ein Silberhalogenidentwicklungsinhibitor, n = 0 oder 1, und das durch TIME dargestellte Bindeglied eine Gruppe, die nach Abspaltung aus der Kupplungsstelle des Kupplers bei dessen Kupplung mit dem Oxidationsprodukt des Silberhalogenidentwicklungsmittels befähigt ist, in einer Folgereaktion den daran gebundenen Inhibitor freizusetzen. Die Gruppe TIME wird auch als Zeitsteuerglied bezeichnet, weil bei Anwesenheit einer solchen Gruppe der daran gebundene Inhibitor in vielen Fällen verzögert freigesetzt wird und wirksam werden kann. Bekannte Zeitsteuerglieder sind beispielsweise eine Gruppe

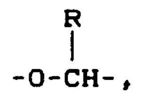

wobei das O-Atom an die Kupplungsstelle des Kupplers und das C-Atom an ein N-Atom eines Inhibitors gebunden ist (z.B. DE-A-27 03 145), eine Gruppe, die nach Abspaltung vom Kuppler einer intramolekularen nukleophilen Verdrängungsreaktion unterliegt und hierbei den Inhibitor freisetzt (z.B. DE-A-28 55 697), eine Gruppe, in der nach Abspaltung vom Kuppler eine Elektronenübertragung entlang eines konjugierten Systems stattfinden kann, wodurch der Inhibitor freigesetzt wird (z.B. DE-A-31 05 026), oder eine Gruppe

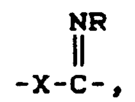

worin X (z.B. -O-) an die Kupplungsstelle des Kupplers und das C-Atom an ein Atom des Inhibitors gebunden ist und worin R beispielsweise für Aryl steht (z.B. EP-A-0 127 063).

TIME kann auch eine Gruppe RED darstellen, die nach Abspaltung vom Kuppler mit dem Oxidationsprodukt des Entwicklers eine Redoxreaktion eingehen kann und als Folge davon die Gruppe INH freisetzt (z.B. EP-A-0 204 175).

Die Gruppe TIME kann vorhanden sein oder auch (im Fall n = 0) völlig fehlen.

Bei der durch Y dargestellten abspaltbaren Gruppe handelt es sich beispielsweise um eine organische Gruppe, die in der Regel über ein Schwefel- oder Stickstoffatom an die Kupplungsstelle des Kupplermoleküls (gegebenenfalls über eine Gruppe TIME) angeknüpft ist. Falls es sich bei der abspaltbaren Gruppe um eine cyclische Gruppe handelt, kann die Anknüpfung an die Kupplungsstelle des Kupplermoleküls (oder an die Gruppe TIME) entweder direkt über ein Atom, das Bestandteil eines Ringes ist, z.B. ein Stickstoffatom, oder indirekt über ein zwischengeschaltetes Bindeglied erfolgt sein. Derartige abspaltbare Gruppen sind in großer Zahl bekannt.

Hierbei handelt es sich durchweg um 5- oder 6-gliedrige heterocyclische Ringe, die über ein Ringstickstoffatom oder ein Schwefelatom mit der Kupplungsstelle des Kupplers oder der Gruppe TIME verbunden sind. Die heterocyclischen Ringe enthalten vielfach benachbart zu dem die Bindung an das Kupplermolekül vermittelnden Stickstoffatom aktivierende Gruppen, z.B. Carbonyl- oder Sulfonylgruppen oder Doppelbindungen.

Wenn die abspaltbare Gruppe über ein Schwefelatom an die Kupplungsstelle des Kupplers gebunden ist, kann es sich bei ihr um den Rest einer diffusionsfähigen Mercaptoverbindung handeln, die die Entwicklung von Silberhalogenid zu inhibieren vermag. Derartige Inhibitorreste sind vielfach als an die Kupplungsstelle von Kupplern, auch offenkettigen Ketomethylenkupplern gebundene abspaltbare Gruppe beschrieben worden, z.B. in US-A-3 227 554.

Bei der abspaltbaren Gruppe handelt es sich beispielsweise um den Rest einer fotografisch wirksamen Verbindung, die einen 5-gliedrigen heterocyclischen Ring enthält, der über ein Schwefelatom an die Kupplungsstelle des Kupplers oder an TIME gebunden ist (Beispiel: Tetrazolylthio, Oxadiazolylthio, Thiadiazolylthio) oder um den Rest einer fotografisch wirksamen Verbindung, die über ein Stickstoffatom eines 1,2,3- oder 1,2,4-Triazolringes an die Kupplungsstelle des Kupplers oder an das Zeitsteuerglied TIME gebunden ist, wobei der 1,2,3-Triazolring einen Benzolring ankondensiert enthalten kann.

In einer bevorzugten Ausführungsform entspricht der DIR-Kuppler der allgemeinen Formel II

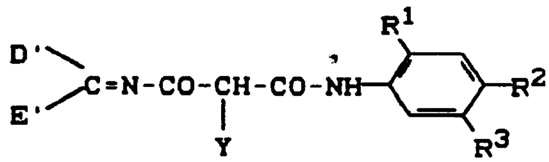

(II)

worin bedeuten

D′ und E′ zusammen einen Rest zur Vervollständigung eines heterocyclischen Ringes, an den ein Benzolring ankondensiert sein kann,

$R^1$ Wasserstoff, Halogen, Alkoxy oder Aroxy;

$R^2$ Wasserstoff, Alkoxy, Alkoxycarbonyl;

$R^3$ Wasserstoff, Sulfamoyl, N-Alkylsulfamoyl, N-Acylsulfamoyl, Alkoxycarbonyl, Acylamino;

Y einen bei Farbkupplung freisetzbaren Rest mit Silberhalogenidentwicklungsinhibierungsfunktion.

Beispiele für erfindungsgemäße DIR-Kuppler sind im folgenden angegeben:

DIR-1

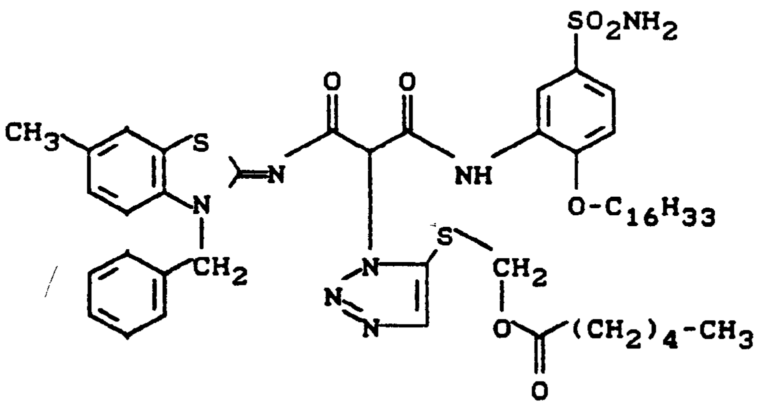

DIR-2

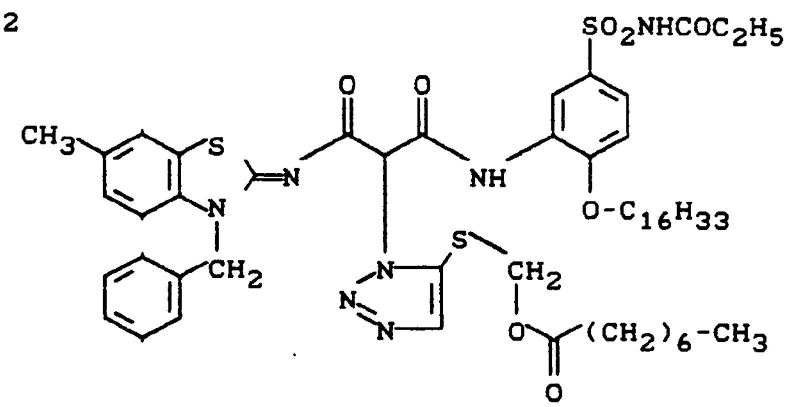

DIR-3

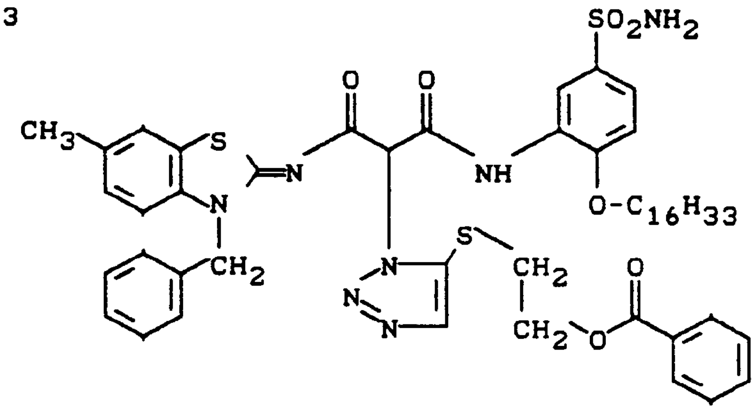

DIR-4

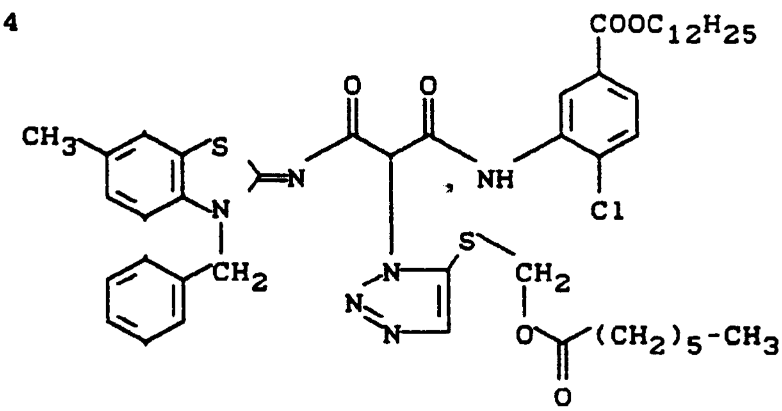

DIR-5

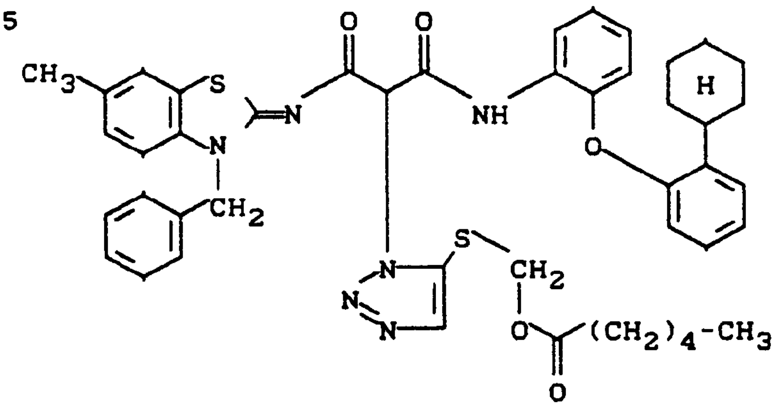

DIR-6

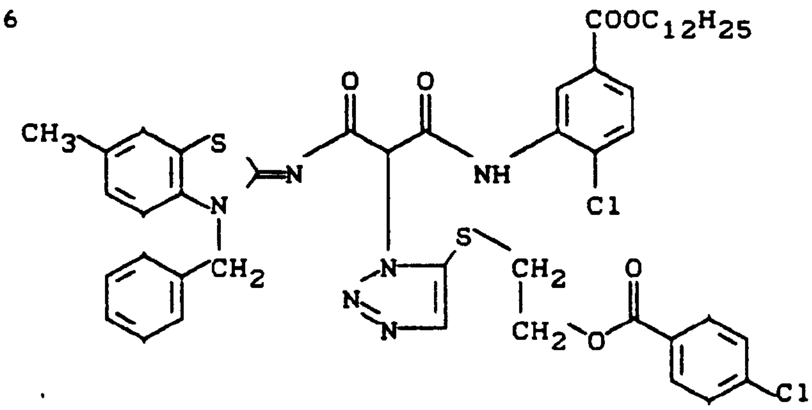

DIR-7

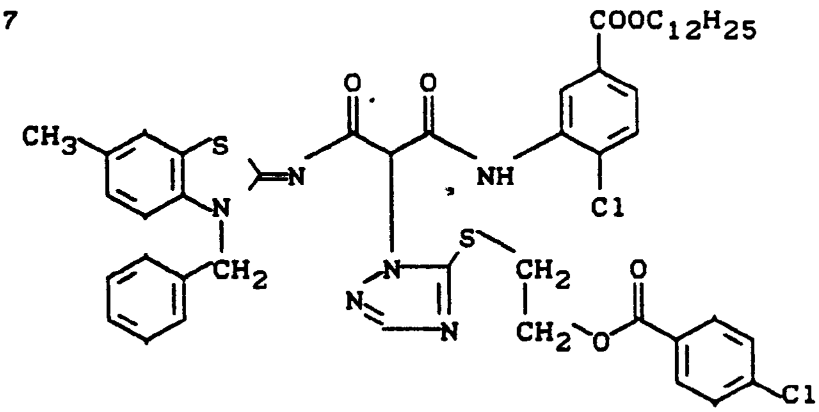

DIR-8

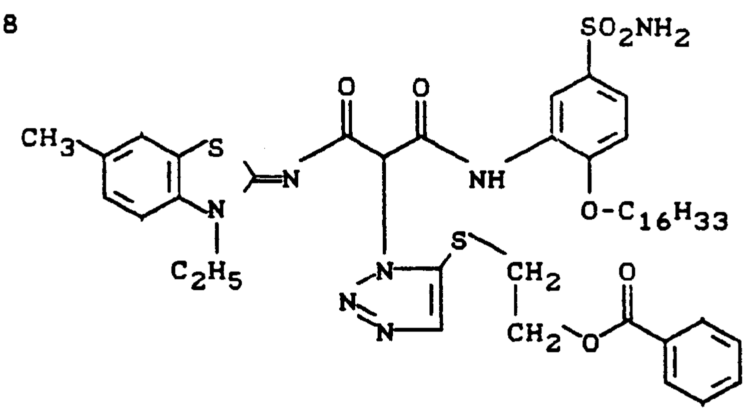

DIR-9

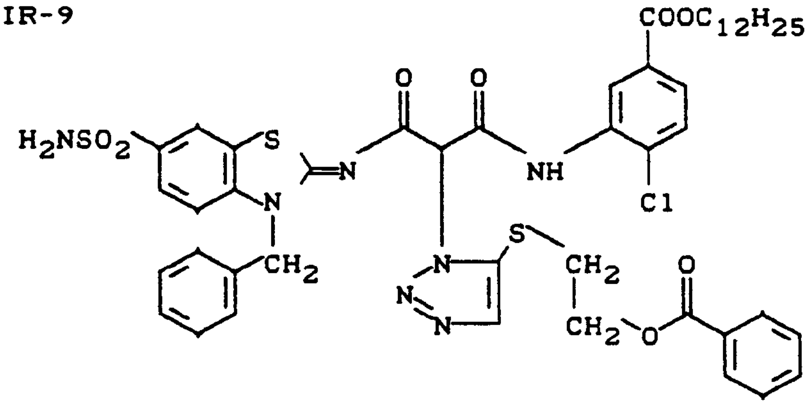

DIR-10

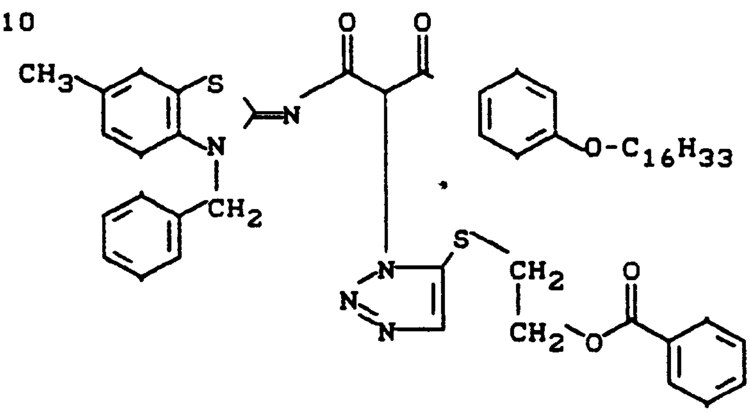

DIR-11

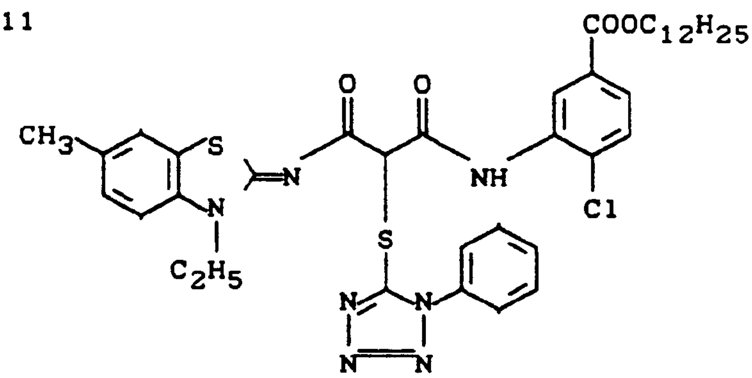

DIR-12

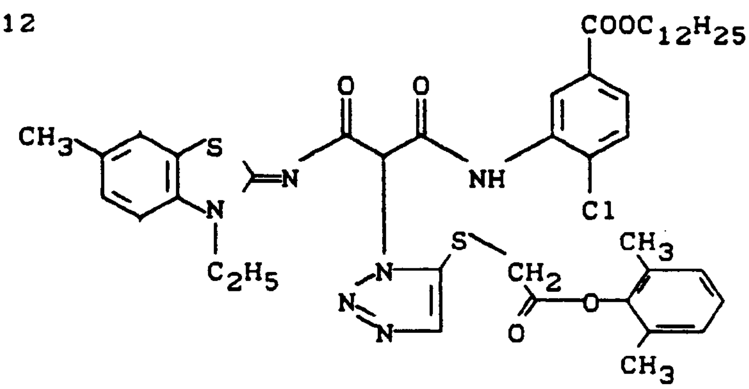

DIR-13

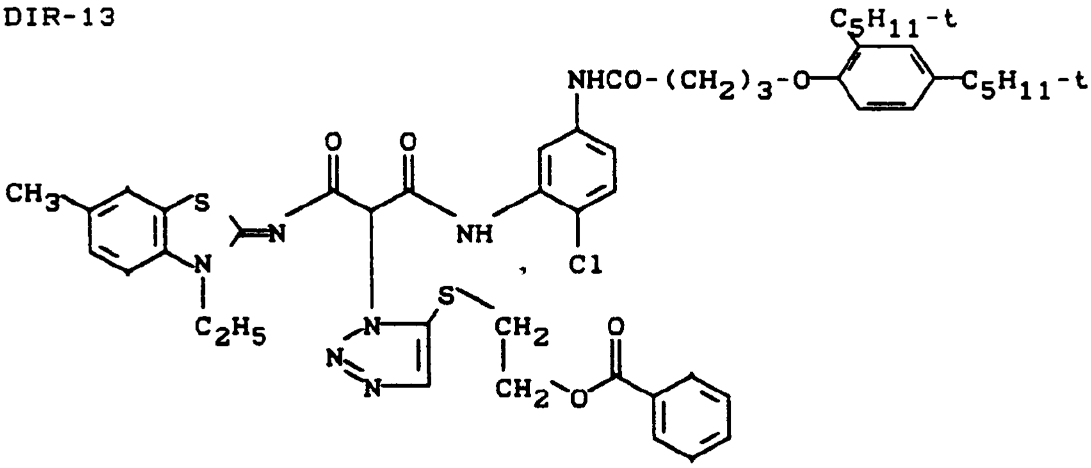

DIR-14

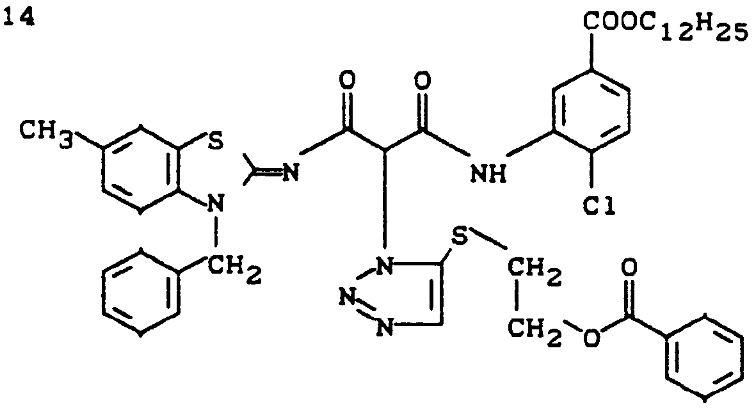

DIR-15

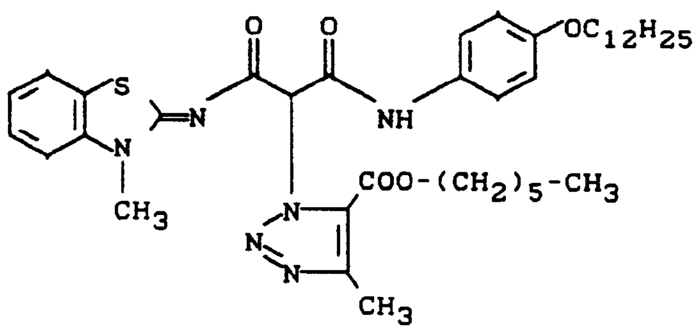

DIR-16

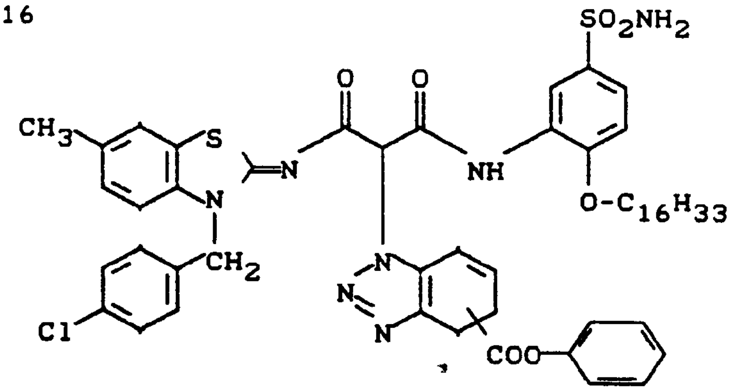

DIR-17

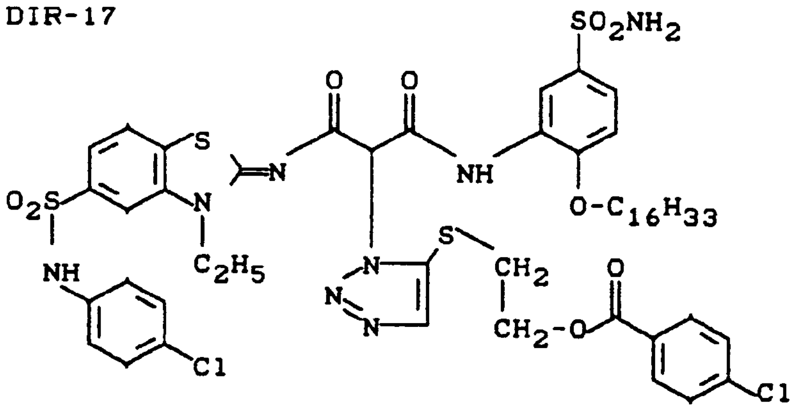

DIR-18

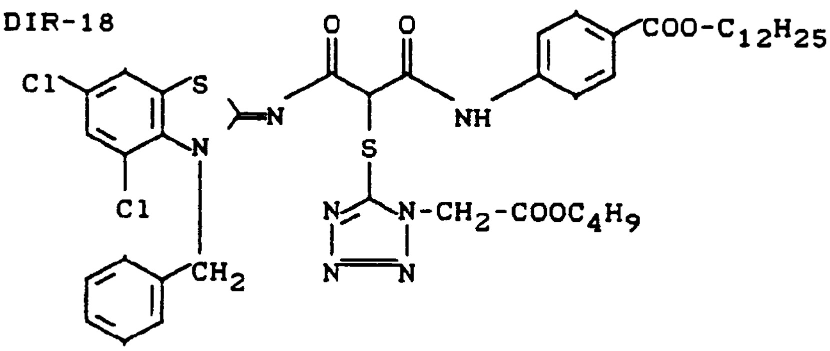

DIR-19

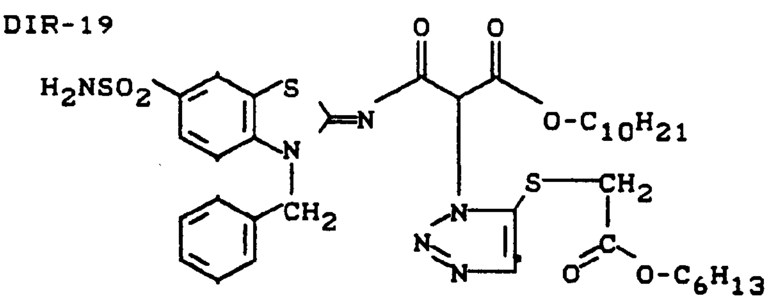

DIR-20

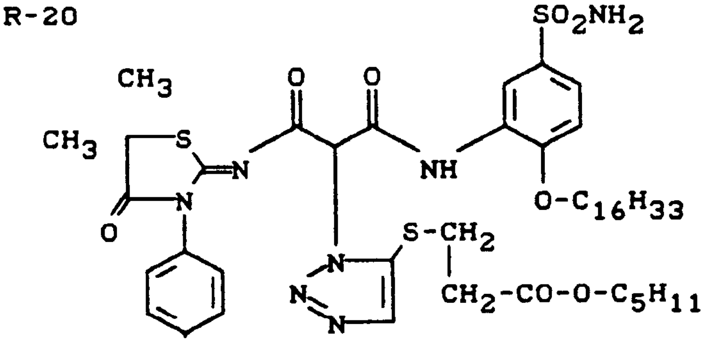

DIR-21

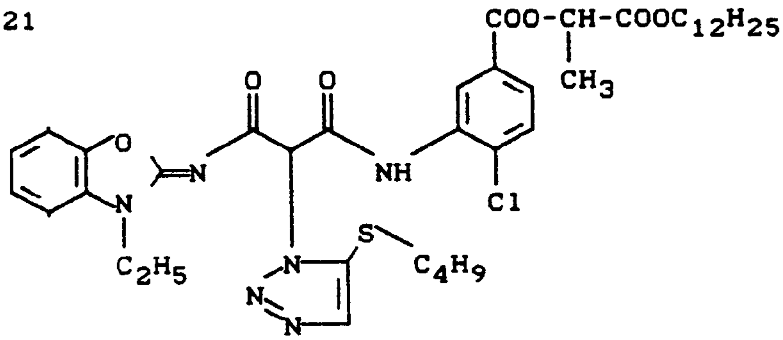

DIR-22

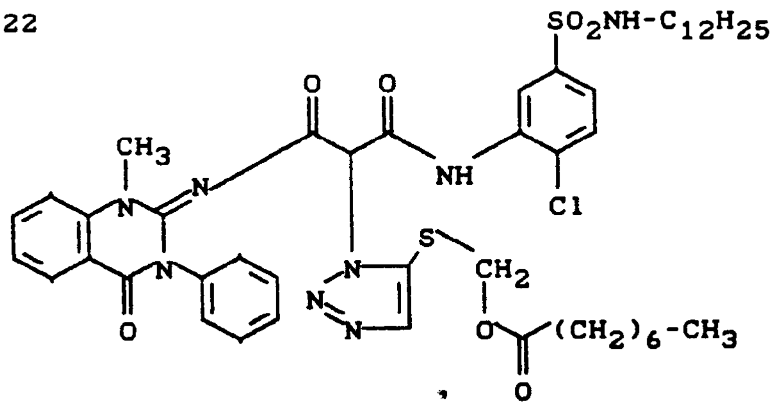

DIR-23

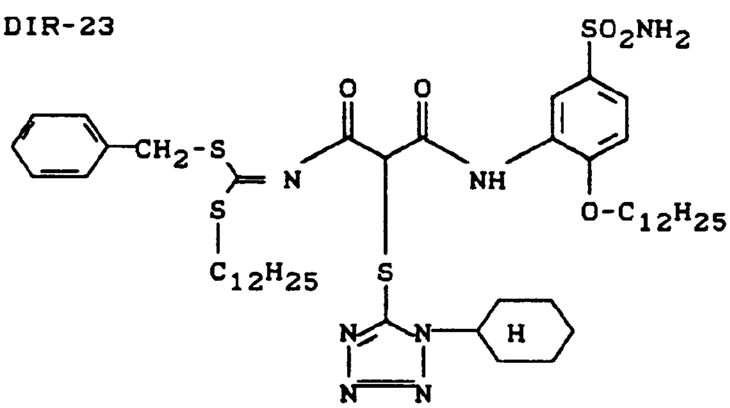

DIR-24

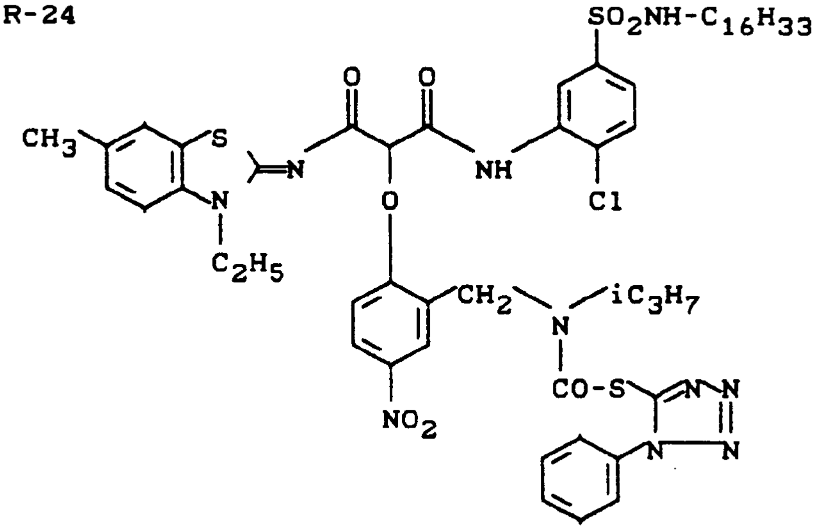

DIR-25

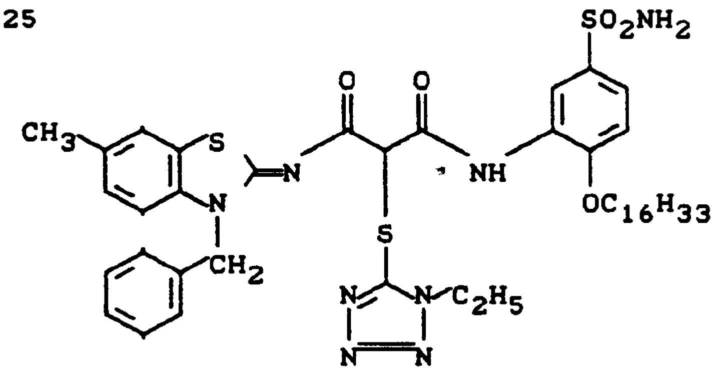

DIR-26

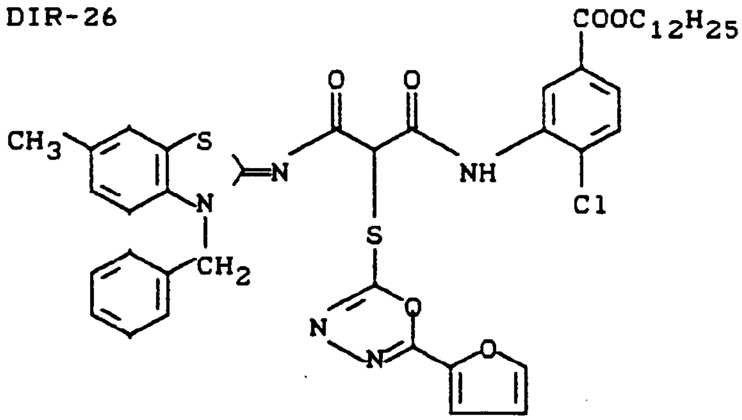

DIR-27

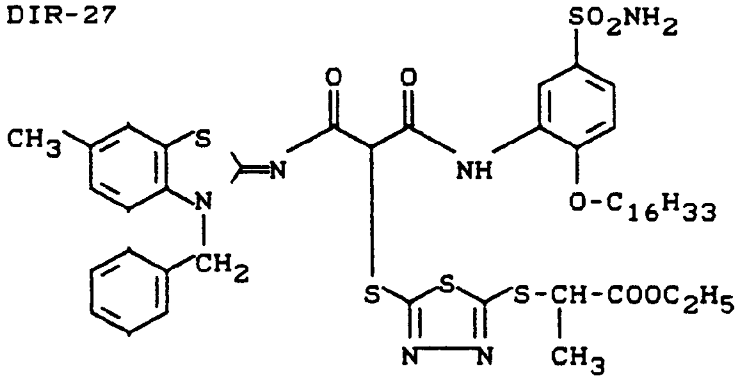

DIR-28

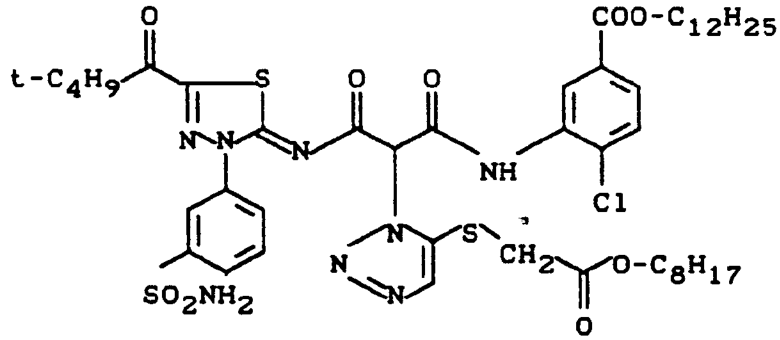

DIR-29

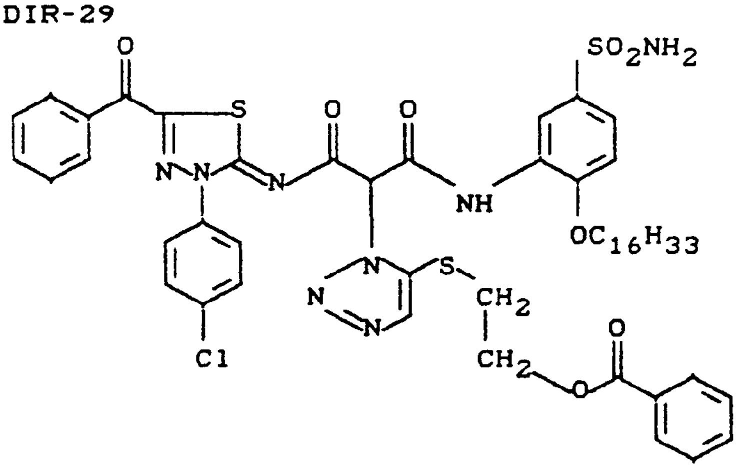

DIR-30

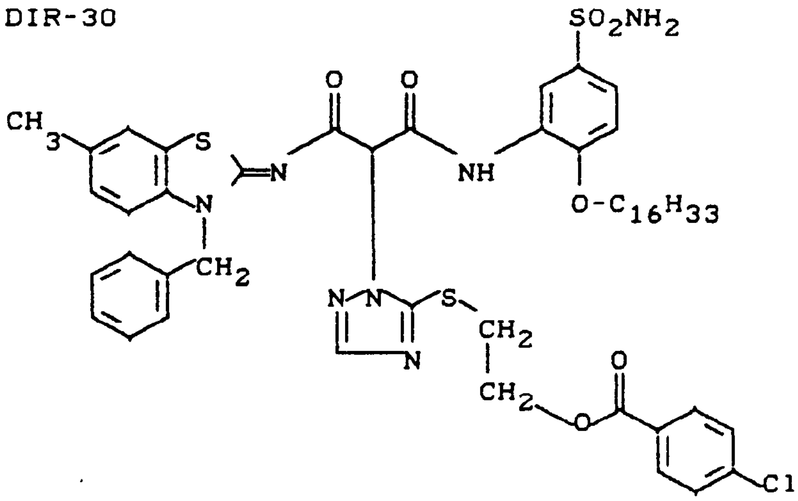

DIR-31

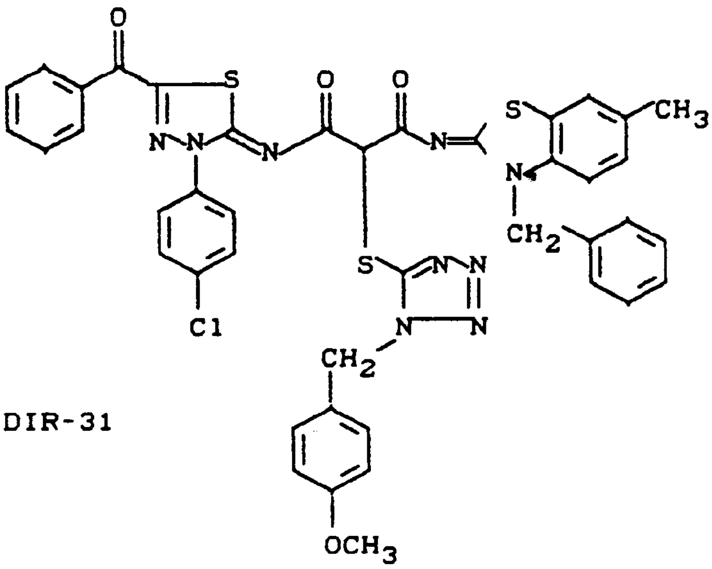

DIR-32

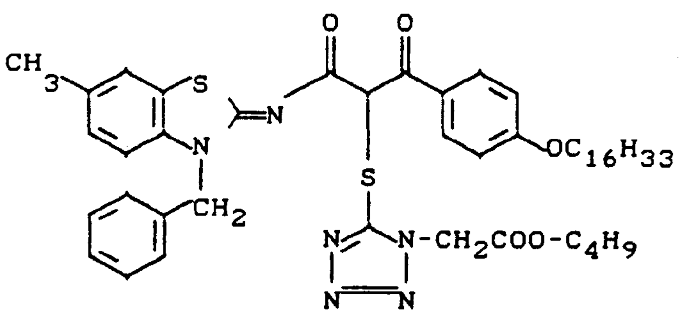

DIR-33

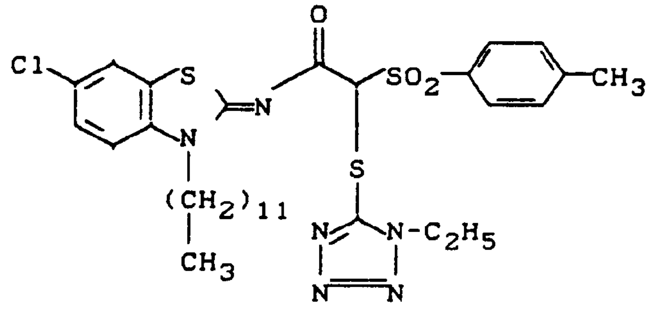

DIR-34

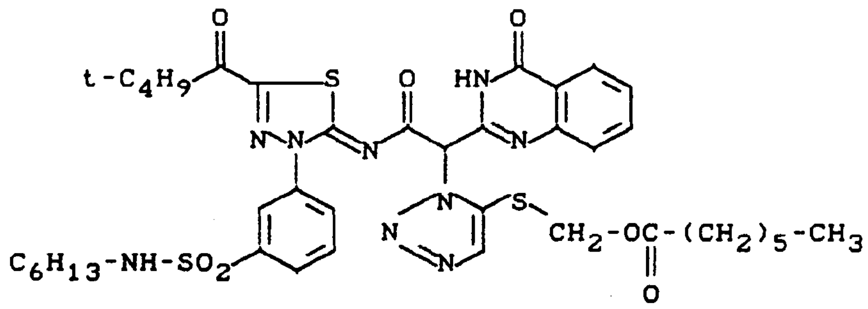

Herstellung von DIR-Kuppler DIR-1

A) 4-(n-Pentylcarbonyloxymethylthio)-1,2,3-triazol

17,2 g 4-Mercapto-1,2,3-triazol-Natriumsalz in 100 ml Dimethylformamid werden innerhalb von 30 min mit 23,1 g Hexansäurechlormethylester unter Rühren versetzt. Nach 2 h wird das Gemisch zu 600 ml Eiswasser gegeben und das entstehende Öl in Ethylacetat aufgenommen.

Die organische Phase wird abgetrennt und nacheinander mit verdünnter $NaHCO_3$-Lösung, 2 n HCl und Wasser gewaschen und danach über $Na_2SO_4$ getrocknet. Nach Entfernen des Lösungsmittels erhält man 22 g des Rohproduktes A als Öl. Daraus werden mit 200 ml Petrolether unter Kühlung 13,2 g der Verbindung A kristallin erhalten.

B) 3-Benzyl-6-methylbenzthiazolonimin

23,4 g N-Benzyl-4-toluidin Hydrochlorid und 19,4 g Kaliumrhodonid in 200 ml Eisessig werden bei 15 - 20°C unter Rühren innerhalb von 45 min mit 5,15 ml Brom in 25 ml Eisessig versetzt.

Nach weiteren 30 min wird der Niederschlag abgetrennt und mit Eisessig, danach mit Eiswasser und Acetonitril gewaschen.

Aus dem Niederschlag wird mit einem geringen Überschuß NaOH die freie Iminoverbindung B erhalten. Ausbeute: 14,5 g.

C) α-Ethoxycarbonyl-2-hexadecyloxy-5-sulfamoyl-acetanilid

238 g 3-Amino-4-cetyloxy-benzolsulfonamid und 102 g Malonsäuremonoethylester-Kaliumsalz in 2.500 ml Ethylacetat werden unter Rühren mit 39 ml Methansulfonsäure versetzt. Danach werden 174 g N,N'-Dicyclohexylcarbodiimid zugegeben und nach weiteren 2 h wird das Reaktionsgemisch auf 60°C erhitzt. Nach Absaugen des Niederschlages wird das Filtrat am Rotavapor eingeengt und der erhaltene Rückstand mit 1.500 ml Methanol verrührt. Der Niederschlag wird abgetrennt und dann mit 500 ml Methanol gewaschen. Ausbeute: 227 g.

D) α-(3-Benzyl-6-methylbenzthiazoloniminocarbonyl)-2'-hexadecyloxy-5'-sulfamoyl-acetanilid

101,8 g der Verbindung B und 210,7 g der Verbindung C werden miteinander vermischt und unter Rühren auf 170 bis 180°C erhitzt. Nach 1 h läßt man auf 150°C abkühlen. 600 ml Dimethylacetamid läßt man langsam zu dem Reaktionsgemisch zulaufen. Die erhaltene Lösung wird auf 30°C gekühlt und mit 2.000 ml Methanol versetzt. Nach Kühlen auf 10°C wird der erhaltene Niederschlag abgetrennt und mit 1.000 ml Methanol gewaschen. Ausbeute: 244 g.

E) Bromierung von D

29 g der Verbindung D werden in 250 ml Eisessig bei 50 - 60°C mit 2,04 ml Brom versetzt. Nach 15

min wird auf Raumtemperatur abgekühlt und mit 200 ml Methanol versetzt. Der entstandene Niederschlag wird abgetrennt und mit Methanol gewaschen. Ausbeute: 24 g.

Verbindung DIR-1

Nacheinander werden 40,7 g der Verbindung E und 11,46 g der Verbindung A in 300 ml Dimethylacetamid gelöst und danach das Gemisch mit 13 ml Tetramethylguanidin versetzt. Nach 1,5 h Rühren bei Raumtemperatur wird das Gemisch auf Eiswasser gegeben, dem vorher zur Neutralisation Eisessig zugegeben wurde.

Der entstehende Niederschlag wird auf einer Nutsche abgesaugt und nacheinander mit Wasser und Methanol gewaschen.

Das erhaltene Rohprodukt (38,7 g) wird aus 800 ml eines Gemisches aus Methanol und Essigester (im Verhältnis 8 : 2) unter Zusatz von A-Kohle umkristallisiert. Ausbeute: 22,3 g.

Mit den erfindungsgemäßen DIR-Kupplern werden ausgezeichnete Interimage- und Kanteneffekte erzielt, die zu hervorragender Farbwiedergabe und Detailwiedergabe führen. Anders als DIR-Kuppler vom Typ der üblichen Acylacetanilidstruktur zeigen sie hohe Lagerstabilität bei Feucht- und/oder Hitzelagerung. Bevorzugte DIR-Kuppler sind solche, deren Inhibitor nach Abspaltung vom Kuppler im Entwickler nach kurzer Zeit zu nicht weiter fotografisch wirksamen Produkten zersetzt wird.

Die meisten DIR-Kuppler weisen $pK_a$-Werte zwischen 10,5 und 7,0 auf, (Bestimmung in Dimethylsulfoxid mit Tetrabutylammoniumhydroxid). Für spezielle Effekte, z.B. wenn hauptsächlich die Körnigkeit beeinflußt werden soll, sind auch $pK_a$-Werte außerhalb des genannten Bereiches geeignet.

Die hohe Wirksamkeit dieser DIR-Kuppler besteht darin, daß sie während der Entwicklung den Entwicklungsinhibitor in ausreichender Menge und mit ausreichender Geschwindigkeit freisetzen.

Es ist klar, daß diese Eigenschaften auch von Kupplern gefordert werden, die eine andere fotografisch wirksame Gruppe abspalten sollen, z.B. eine entwicklungsbeschleunigende oder eine verschleiernde Gruppe, die beide ebenfalls nur in der Phase der Entwicklung wirksam werden können.

Demnach kann die Gruppe nicht nur die Bedeutung eines Entwicklungsinhibitorrestes sondern auch einer anderen fotografisch wirksamen Gruppe haben.

Kuppler mit Hetarylideniminogruppen waren bereits bekannt, z.B. US-A-2 312 040 und Bull. Soc. Chem. Belg. 61 (1952), S. 245 - 252.

Diese Kuppler sind 4-Äquivalentkuppler und haben bisher in fotografischen Materialien offensichtlich keine Verwendung gefunden.

Ein Hinweis darauf, daß diese Kupplerteilstrukturen geeignet sein könnten, um als 2-Äquivalentkuppler fotografisch wirksame Gruppen abzuspalten, ist den beiden Publikationen nicht zu entnehmen.

Die Verbindungen der vorliegenden Erfindung eignen sich für die Verwendung als DIR-Kuppler in farbfotografischen, insbesondere mehrschichtigen Aufzeichnungsmaterialien. Wenn es sich um Gelbkuppler handelt, werden sie bevorzugt in oder zugeordnet zu einer lichtempfindlichen Silberhalogenidemulsionsschicht mit einer überwiegenden Empfindlichkeit für den blauen Spektralbereich des sichtbaren Lichtes verwendet. Der besondere Vorteil der erfindungsgemäßen Kuppler, nämlich eine vergleichsweise geringe Entwicklungsinhibierung in der Schicht, der eine solche Verbindung zugeordnet ist, neben einer vergleichsweise hohen Entwicklungsinhibierung in benachbarten nicht zugeordneten Schichten, kommt naturgemäß besonders dann zum Tragen, wenn es sich um ein mehrschichtiges farbfotografisches Aufzeichnungsmaterial handelt, das neben einer überwiegend blauempfindlichen Silberhalogenidemulsionsschicht weitere lichtempfindliche Silberhalogenidemulsionsschichten enthält mit überwiegender Empfindlichkeit für den grünen bzw. roten Spektralbereich des sichtbaren Lichtes.

Die erfindungsgemäßen DIR-Kuppler können wegen ihrer außerordentlich hohen Wirksamkeit in vergleichsweise geringen Mengen eingesetzt werden um die erwünschten Effekte, insbesondere die Interimageeffekte hervorzubringen. Dies ermöglicht es beispielsweise, einen erfindungsgemäßen DIR-Kuppler nicht nur in den blauempfindlichen Gelbfarbstoff erzeugenden Schichten sondern auch in anderen Schichten einzusetzen, ohne daß dort eine zu hohe unerwünschte Nebendichte auftritt. Die erfindungsgemäßen DIR-Kuppler sind somit als Gelbkuppler mit Vorteil auch in Purpurschichten wie auch in Blaugrünschichten anwendbar.

Bei der Herstellung des lichtempfindlichen farbfotografischen Aufzeichnungsmaterials können die diffusionsfesten DIR-Kuppler der vorliegenden Erfindung gegebenenfalls zusammen mit anderen Kupplern in bekannter Weise in die Gießlösung der Silberhalogenidemulsionsschichten oder anderer Kolloidschichten eingearbeitet werden. Beispielsweise können öllösliche oder hydrophobe Kuppler vorzugsweise aus einer Lösung in einem geeigneten Kupplerlösungsmittel (Ölbildner) gegebenenfalls in Anwesenheit eines Netz- oder Dispergiermittels zu einer hydrophilen Kolloidlösung zugefügt werden. Die hydrophile Gießlösung kann selbstverständlich neben dem Bindemittel andere übliche Zusätze enthalten. Die Lösung des Kupplers braucht nicht direkt in die Gießlösung für die Silberhalogenidemulsionsschicht oder eine andere wasserdurchlässige Schicht

dispergiert zu werden; sie kann vielmehr auch vorteilhaft zuerst in einer wäßrigen nichtlichtempfindlichen Lösung eines hydrophilen Kolloids dispergiert werden, worauf das erhaltene Gemisch gegebenenfalls nach Entfernung der verwendeten niedrig siedenden organischen Lösungsmittel mit der Gießlösung für die lichtempfindliche Silberhalogenidemulsionsschicht oder einer anderen wasserdurchlässigen Schicht vor dem Auftragen vermischt wird.

Als lichtempfindliche Silberhalogenidemulsionen eignen sich Emulsionen von Silberchlorid, Silberbromid oder Gemischen davon, evtl. mit einem Gehalt an Silberiodid bis zu 20 mol-% in einem der üblicherweise verwendeten hydrophilen Bindmittel. Als Bindemittel für die fotografischen Schichten wird vorzugsweise Gelatine verwendet. Diese kann jedoch ganz oder teilweise durch andere natürliche oder synthetische Bindemittel ersetzt werden.

Die Emulsionen können in der üblichen Weise chemisch und spektral sensibilisiert sein, und die Emulsionsschichten wie auch andere nicht-lichtempfindliche Schichten können in der üblichen Weise mit bekannten Härtungsmitteln gehärtet sein.

Üblicherweise enthalten farbfotografische Aufzeichnungsmaterialen mindestens je eine Silberhalogenidemulsionsschicht für die Aufzeichnung von Licht der drei Spektralbereiche Rot, Grün und Blau. Zu diesem Zweck sind die lichtempfindlichen Schichten in bekannter Weise durch geeignete Sensibilisierungsfarbstoffe spektral sensibilisiert. Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtemfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-25 30 645, DE-A-26 22 922).

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, hier besonders Farbkuppler, zur Erzeugung der unterschiedlichen Teilfarbenbilder Blaugrün, Purpur und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogendemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (z.B. Blaugrün, Purpur, Gelb) zugeordnet ist.

Jeder der unterschiedlich spektral sensibilisierten Silberhalogenidemulsionsschichten kann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Bei bevorzugten Ausführungsformen sind rotempfindlichen Silberhalogenidemulsionsschichten mindestens ein nichtdiffundierender Farbkuppler zur Erzeugung des blaugrünen Teilfarbenbildes, grünempfindlichen Silberhalogenidemulsionsschichten mindestens ein nichtdiffundierender Farbkuppler zur Erzeugung des purpurnen Teilfarbenbildes und blauempfindlichen Silberhalogenidemulsionsschichten mindestens ein nichtdiffundierender Farbkuppler zur Erzeugung des gelben Teilfarbenbildes zugeordnet.

Es sind aber auch andere Zuordnungen bekannt.

Farbkuppler zur Erzeugung des blaugrünen Teilfarbenbildes sind in der Regel Kuppler vom Phenol- oder α-Naphtholtyp; geeignete Beispiele hierfür sind

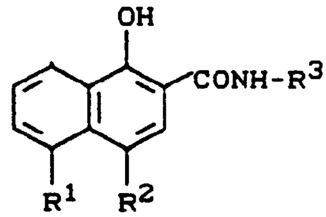

C-1: $R^1$, $R^2$ = H;

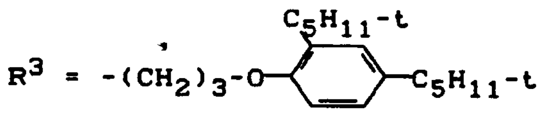

C-2: $R^1$ = $-NHCOOCH_2-CH(CH_3)_2$; $R^2$ = H;
$R^3$ = $-(CH_2)_3-OC_{12}H_{25}$

C-3: $R^1$ = H; $R^2$ = $-OCH_2-CH_2-SO_2CH_3$; $R^3$ =$-C_{16}H_{33}$

C-4: $R^1$ = H; $R^2$ = $-OCH_2-CONH-(CH_2)_2-OCH_3$;

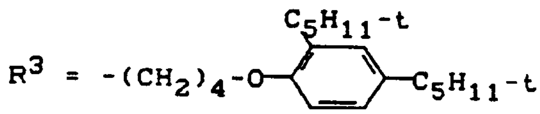

C-5: $R^1$, $R^2$ = H;

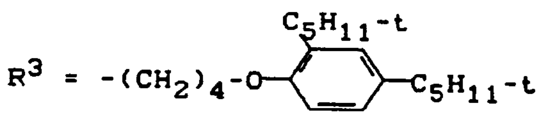

C-6: $R^1$, $R^2$ = H;

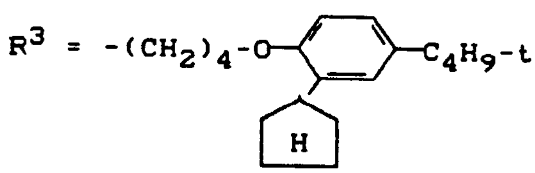

C-7: $R^1$ = H; $R^2$ = Cl; $R^3$ = $-C(C_2H_5)_2-C_{21}H_{43}$

C-8: $R^1$ = H; $R^2$ = $-O-CH_2-CH_2-S-CH(COOH)-C_{12}H_{25}$
$R^3$ = Cyclohexyl

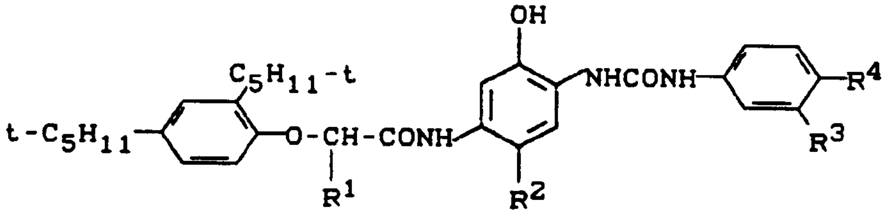

C-9: $R^1 = -C_4H_9$; $R^2 = H$; $R^3 = -CN$; $R^4 = Cl$
C-10: $R^1 = -C_4H_9$; $R^2 = H$; $R^3 = H$; $R^4 = -SO_2CHF_2$
C-11: $R^1 = -C_4H_9$;

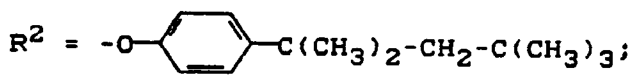

$R^3 = H$; $R^4 = -CN$
C-12: $R^1 = C_2H_5$; $R^2, R^3 = H$; $R^4 = -SO_2CH_3$
C-13: $R^1 = -C_4H_9$; $R^2, R^3 = H$; $R^4 = -SO_2-C_4H_9$
C-14: $R^1 = -C_4H_9$; $R^2 = H$; $R^3 = -CN$; $R^4 = -CN$
C-15: $R^1 = -C_4H_9$; $R^2, R^3 = H$; $R^4 = -SO_2-CH_2-CHF_2$
C-16: $R^1 = -C_2H_5$; $R^2, R^3 = H$; $R^4 = -SO_2CH_2-CHF-C_3H_7$
C-17: $R^1 = -C_4H_9$; $R^2, R^3 = H$; $R^4 = F$
C-18: $R^1 = -C_4H_9$; $R^2, R^3 =H$; $R^4 = -SO_2CH_3$
C-19: $R^1 = -C_4H_9$; $R^2, R^3 =H$; $R^4 = -CN$

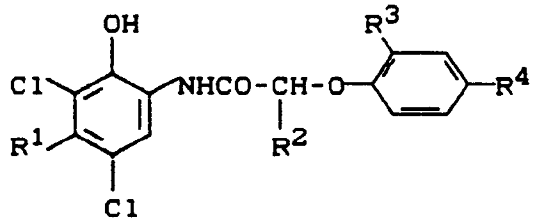

C-20: $R^1 = -CH_3$; $R^2 = -C_2H_5$; $R^3, R^4 = -C_5H_{11}$-t
C-21: $R^1 = -CH_3$; $R^2 = H$; $R^3, R^4 = -C_5H_{11}$-t
C-22: $R^1, R^2 = -C_2H_5$; $R^3, R^4 = -C_5H_{11}$-t
C-23: $R^1 = -C_2H_5$; $R^2 = -C_4H_9$; $R^3, R^4 = -C_5H_{11}$-t
C-24: $R^1 = -C_2H_5$; $R^2 = -C_4H_9$; $R^3, R^4 = -C_4H_9$-t

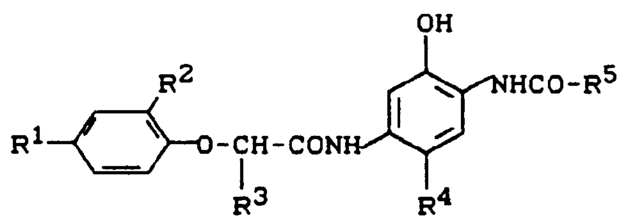

C-25: $R^1, R^2 = -C_5H_{11}$-t; $R^3 = -C_4H_9$; $R^4 = H$; $R^5 = -C_3F_7$
C-26: $R^1 = -NHSO_2-C_4H_9$; $R^2 = H$; $R^3 = -C_{12}H_{25}$; $R^4 = Cl$;
$R^5$ = Phenyl
C-27: $R^1, R^2 = -C_5H_{11}$-t; $R^2 = Cl$, $R^3 = -C_3H_7$-i; $R^4 = Cl$;
$R^5$ = Pentafluorphenyl
C-28: $R^1 = -C_5H_{11}$-t; $R^2 = Cl$; $R^3 = -C_6H_{13}$; $R^4 = Cl$;
$R^5$ = -2-Chlorphenyl

Farbkuppler zur Erzeugung des purpurnen Teilfarbenbildes sind in der Regel Kuppler vom Typ des 5-Pyrazolons, des Indazolons oder der Pyrazoloazole; geeignete Beispiele hierfür sind

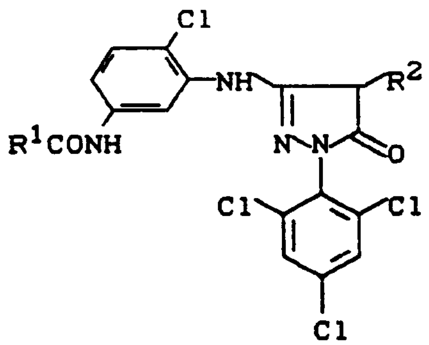

M-1:

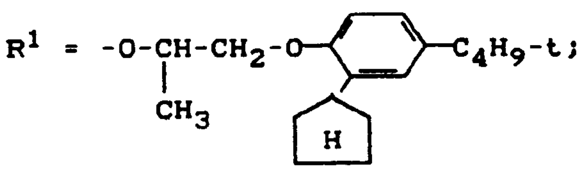

$R^2 = H$

M-2

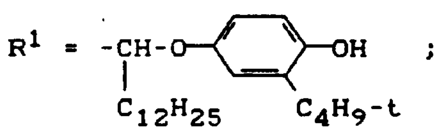

$R^2 = H$

M-3: $R^1 = -C_{13}H_{27}$; $R^2 = H$

M-4: $R^1 = -OC_{16}H_{33}$; $R^2 = H$

M-5: $R^1 = -C_{13}H_{27}$;

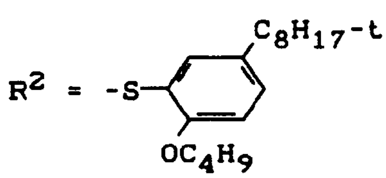

M-6:

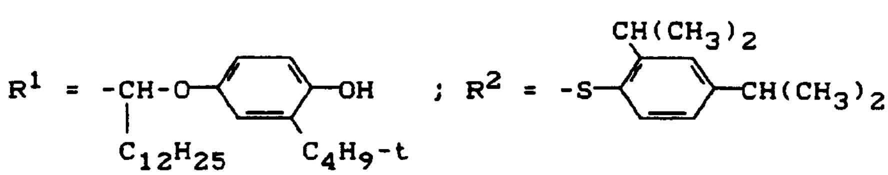

M-7: $R^1 = -C_9H_{19}$;

$R^2$ = 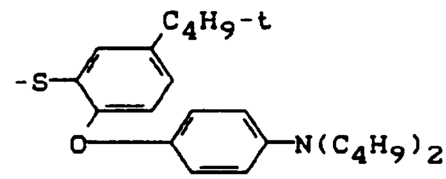

M-8:

$R^1$ = 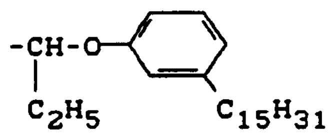 ; $R^2$ = 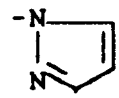

M-9:

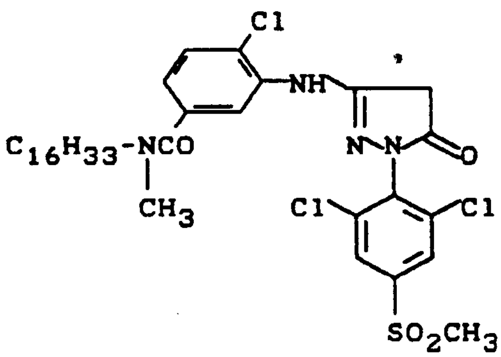

M-10:

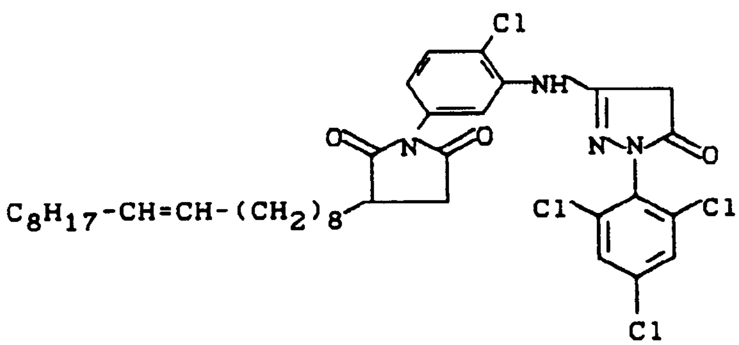

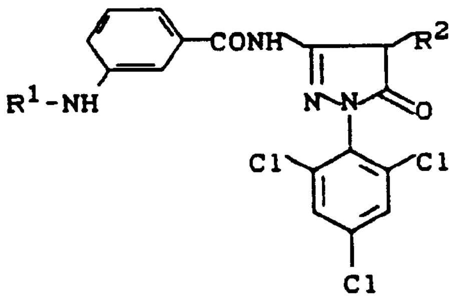

M-11:

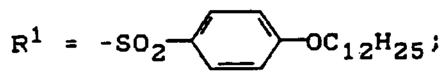

$R^2 = H$

M-12:

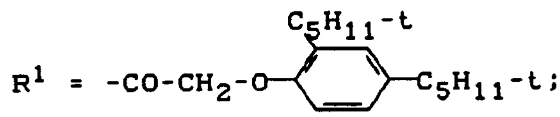

$R^2 = H$

M-13:

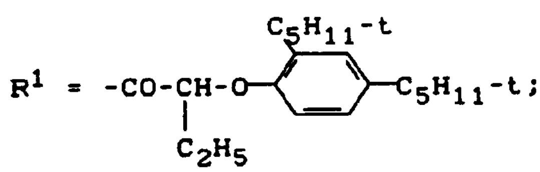

$R^2 = H$

M-14:

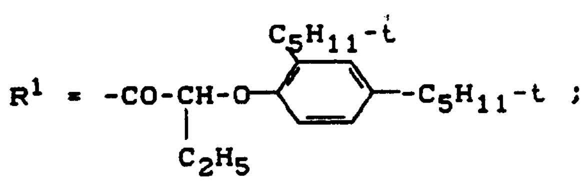

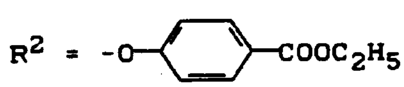

M-15:

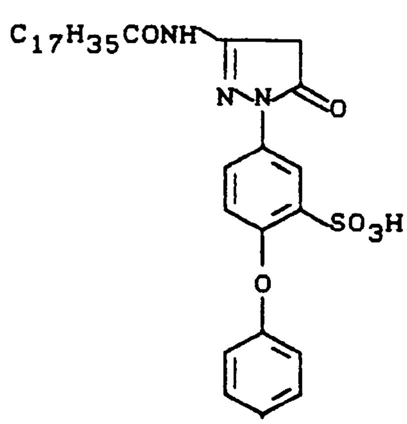

M-16:

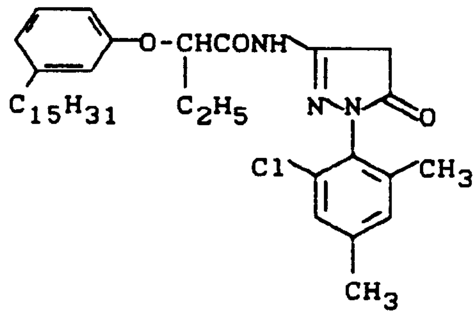

M-17:

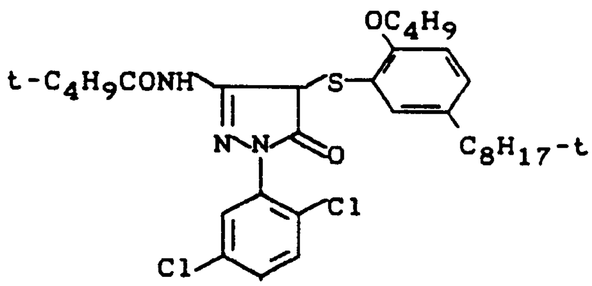

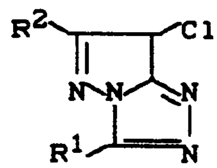

M-18:

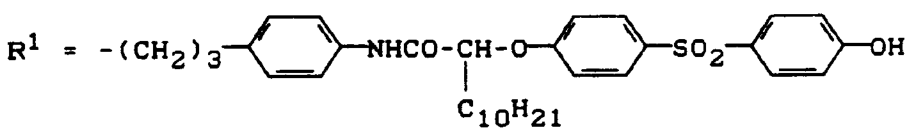

$R^2 = -CH_3$

M-19:

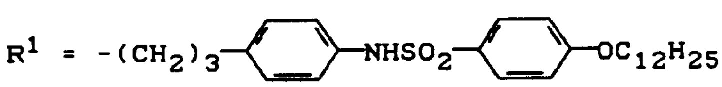

$R^2 = -CH_3$

M-20:

$R^1$ = 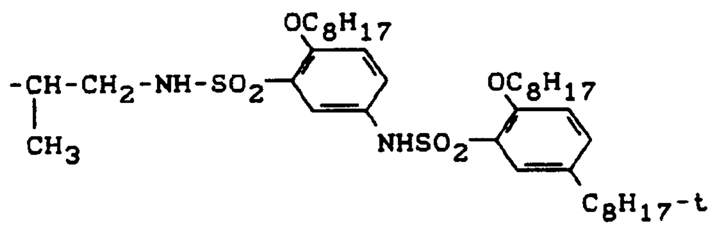

$R^2 = -C_4H_9$-t

M-21:

$R^1$ = 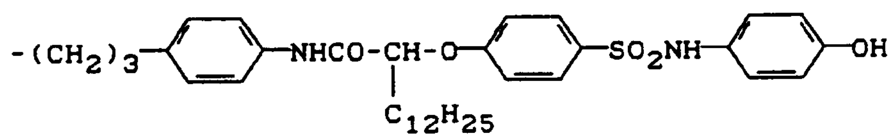

$R^2 = -CH_3$

M-22:

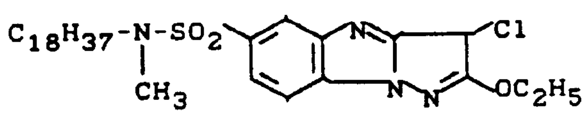

Farbkuppler zur Erzeugung des gelben Teilfarbenbildes sind in der Regel Kuppler mit einer offenkettigen Ketomethylengruppierung, insbesondere Kuppler vom Typ des α-Acylacetamids; geeignete Beispiele hierfür sind α-Benzoylacetanilidkuppler und α-Pivaloylacetanilidkuppler der Formeln

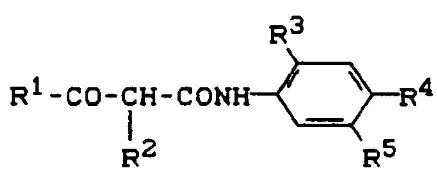

Y-1: $R^1 = -C_4H_9$-t;

$R^2$ = 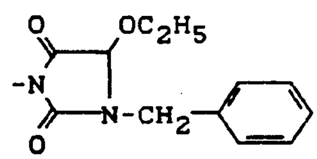 ;

$R^3$ = Cl; $R^4$ = H;

$R^5$ = 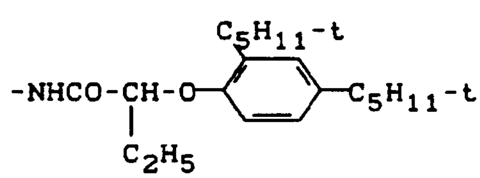

Y-2: $R^1 = -C_4H_9$-t;

$R^2$ = 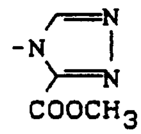 ;

$R^3$ = $-OC_{16}H_{33}$; $R^4$ = H;
$R^5$ = $-SO_2NHCH_3$

Y-3: $R^1$ = $-C_4H_9$-t;

$R^2$ = 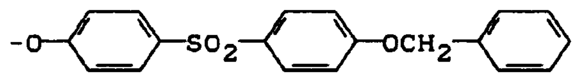 ;

$R^3$ = Cl
$R^4$ = H; $R^5$ = $-NHSO_2-C_{16}H_{33}$

Y-4: $R^1$ = $-C_4H_9$-t;

$R^2$ = 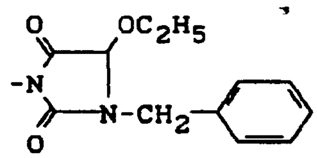 ;

$R^3$ = Cl;
$R^4$ = H; $R^5$ = $-COOC_{12}H_{25}$

Y-5: $R^1$ = $-C_4H_9$-t;

$R^2$ = 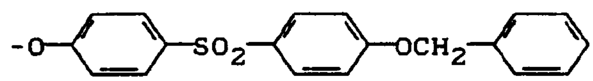 ;

$R^3$ = Cl;
$R^4$ = H;

$R^5$ = 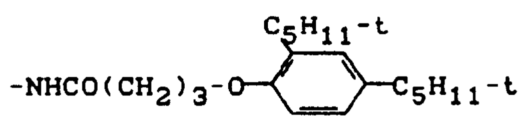

Y-6: $R^1$ = $-C_4H_9$-t;

$R^2$ = 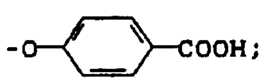

$R^3$ = Cl; $R^4$ = H;

$R^5$ = 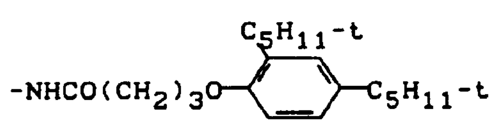

Y-7: $R^1$ = $-C_4H_9$-t;

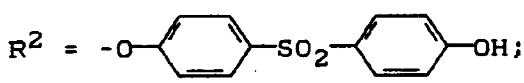

$R^3$ = Cl;
$R^4$ = H; $R^5$ = $-NHSO_2-C_{16}H_{33}$

Y-8: $R^1$ = $-C_4H_9$-t;

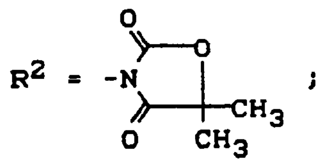

$R^3$ = Cl; $R^4$ = H;

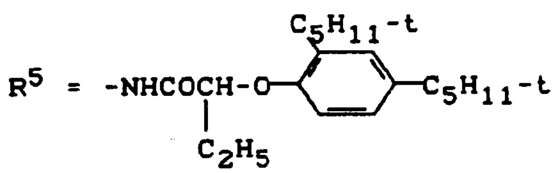

Y-9: $R^1$ = $-C_4H_9$-t;

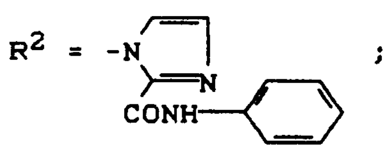

$R^3$ = $-OC_{16}H_{33}$;
$R^4$ = H; $R^5$ = $-SO_2NHCOC_2H_5$

Y-10: $R^1$ = $-C_4H_9$-t;

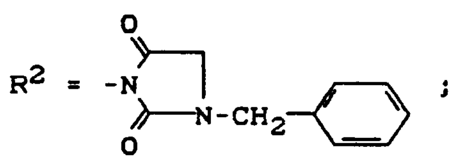

$R^3$ = Cl; $R^4$ = H

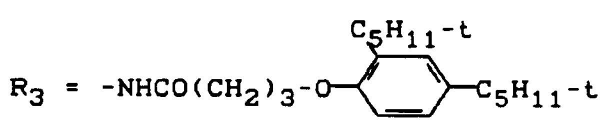

Y-11: $R^1$ = $-C_4H_9$-t;

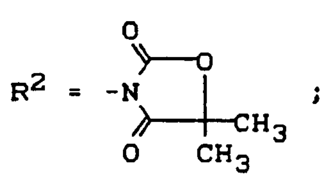

$R^3 = Cl$; $R^4 = H$;

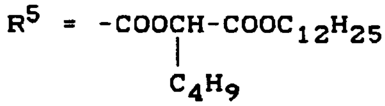

Y-12: $R^1 = -C_4H_9$-t;

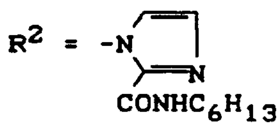
;

$R^3 = Cl$; $R^4 = H$;

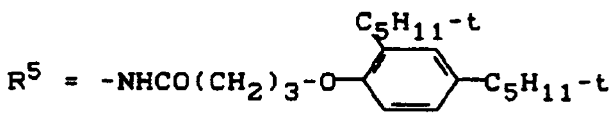

Y-13: $R^1 = -C_4H_9$-t;

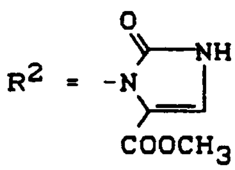
;

$R^3 = -OC_{16}H_{33}$; $R^4 = H$;
$R^5 = -SO_2NHCH_3$

Y-14: $R^1 = -C_4H_9$-t;

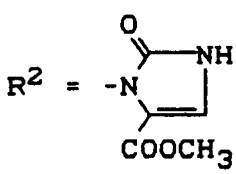
;

$R^3 = Cl$; $R^4 = H$;

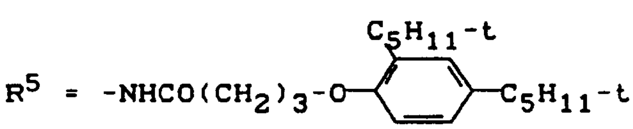

Y-15:

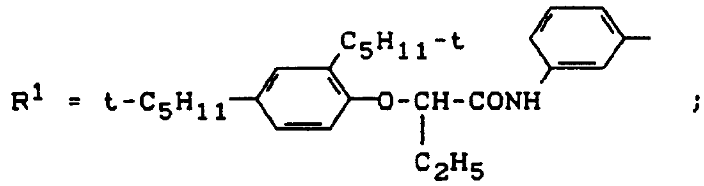

$R^2$, $R^4$, $R^5$ = H; $R^3$ = $-OCH_3$

Y-16:

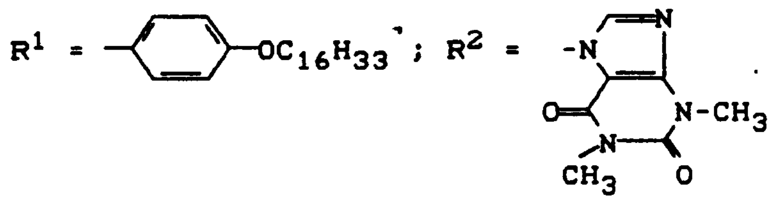

$R^3$, $R^5$ = $-OCH_3$; $R^4$ = H

Y-17:

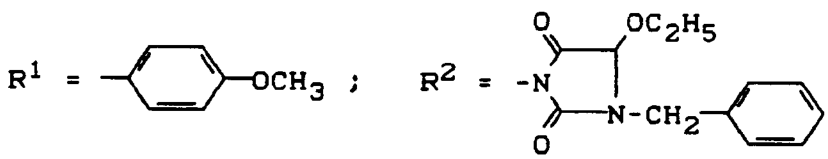

$R^3$ = Cl; $R^4$ = H; $R^5$ = $-COOC_{12}H_{25}$

Y-18:

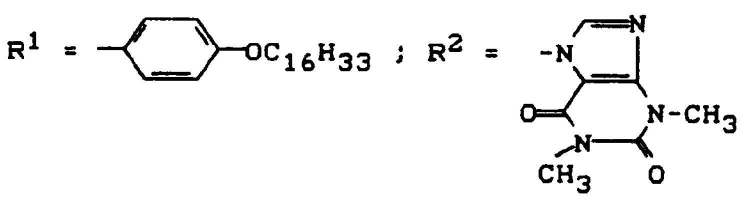

$R^3$ = Cl; $R^4$, $R^5$ = $-OCH_3$

Y-19:

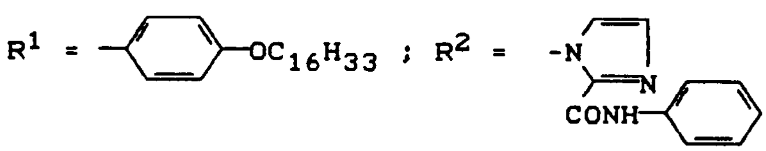

$R^3$ = $-OCH_3$; $R^4$ = H; $R^5$ = $-SO_2N(CH_3)_2$

Y-20:

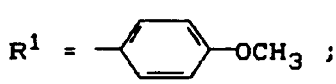

$R^2$ = 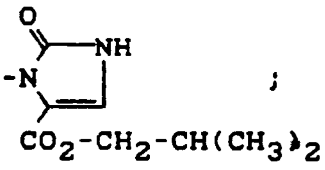 ;

$R^3$ = $-OCH_3$; $R^4$ = H;

$R_5$ = 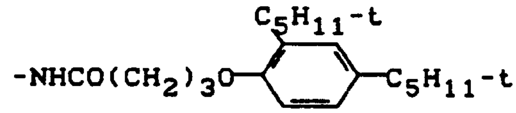

Y-21:

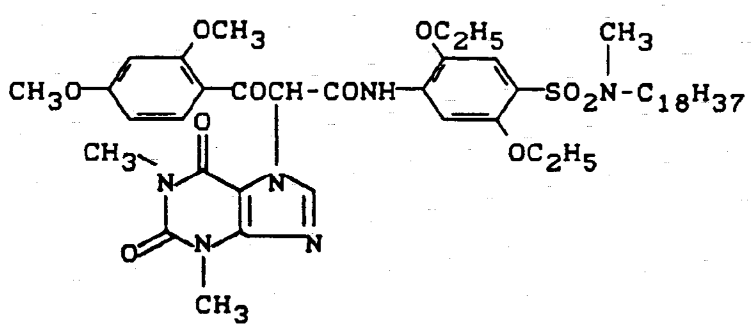

Bei den Farbkupplern kann es sich um 4-Äquivalentkuppler, aber auch um 2-Äquivalentkuppler handeln. Letztere leiten sich von den 4-Äquivalentkupplern dadurch ab, daß sie in der Kupplungsstelle einen Substituenten enthalten, der bei der Kupplung abgespalten wird. Zu den 2-Äquivalentkupplern sind solche zu rechnen, die farblos sind, als auch solche, die eine intensive Eigenfarbe aufweisen, die bei der Farbkupplung verschwindet bzw. durch die Farbe des erzeugten Bildfarbstoffes ersetzt wird (Maskenkuppler), und die Weißkuppler, die bei Reaktion mit Farbentwickleroxidationsprodukten im wesentlichen farblose Produkte ergeben. Zu den 2-Äquivalentkupplern sind ferner solche Kuppler zu rechnen, die in der Kupplungsstelle einen abspaltbaren Rest enthalten, der bei Reaktion mit Farbentwickleroxidationsprodukten in Freiheit gesetzt wird und dabei entweder direkt oder nachdem aus dem primär abgespaltenen Rest eine oder mehrere weitere Gruppen abgespalten worden sind (z.B. DE-A-27 03 145, DE-A-28 55 697, DE-A-31 05 026, DE-A-33 19 428), eine bestimmte erwünschte fotografische Wirksamkeit entfaltet, z.B. als Entwicklungsinhibitor oder -accelerator. Beispiele für solche 2-Äquivalentkuppler sind die bekannten DIR-Kuppler wie auch DAR-bzw. FAR-Kuppler.

Da bei den DIR-, DAR- bzw. FAR-Kupplern hauptsächlich die Wirksamkeit des bei der Kupplung freigesetzten Restes erwünscht ist und es weniger auf die farbbildenden Eigenschaften dieser Kuppler ankommt, kommen auch solche DIR-, DAR- bzw. FAR-Kuppler in Frage, die bei der Kupplung im wesentlichen farblose Produkte ergeben (DE-A-15 47 640).

Der abspaltbare Rest kann auch ein Ballastrest sein, so daß bei der Reaktion mit Farbentwickleroxidationsprodukten Kupplungsprodukte erhalten werden, die diffusionsfähig sind oder zumindest eine schwache bzw. eingeschränkte Beweglichkeit aufweisen (US-A-4 420 556).

Erfindungsgemäß enthält das farbfotografische Aufzeichnungsmaterial zusätzlich mindestens einen DIR-Kuppler der Formel I, wobei dieser Kuppler nicht nur in der Gelbschicht, sondern auch in der Purpurschicht und oder auch in der Blaugrünschicht sowie auch in einer zu einer der genannten Schichten benachbarten nicht lichtempfindlichen Schicht enthalten sein kann.

Über die genannten Bestandteile hinaus kann das farbfotografische Aufzeichnungsmaterial der vorliegenden Erfindung weitere Zusätze enthalten, zum Beispiel Antioxidantien, farbstoffstabilisierende Mittel und Mittel zur Beeinflussung der mechanischen und elektrostatischen Eigenschaften. Um die nachteilige Einwirkung von UV-Licht auf die mit dem erfindungsgemäßen farbfotografischen Aufzeichnungsmaterial hergestellten Farbbilder zu vermindern oder zu vermeiden, ist es vorteilhaft, in einer oder mehreren der in dem Aufzeichnungsmaterial enthaltenen Schichten, vorzugsweise in einer der oberen Schichten, UV-absorbierende Verbindungen

zu verwenden. Geeignete UV-Absorber sind beispielsweise in US-A-3 253 921, DE-C-2 036 719 und EP-A-0 057 160 beschrieben.

Für die erfindungsgemäßen Materialien können die üblichen Schichtträger verwendet werden, siehe Research Disclosure 17 643, Abschnitt XVII.

Als Schutzkolloid bzw. Bindemittel für die Schichten des Aufzeichnungsmaterials sind die üblichen hydrophilen filmbildenden Mittel geeignet, z.B. Proteine, insbesondere Gelatine. Begußhilfsmittel und Weichmacher können verwendet werden. Verwiesen wird auf die in der Research Disclosure 17 643 in Abschnitt IX, XI und XII angegebenen Verbindungen.

Die Schichten des fotografischen Materials können in der üblichen Weise gehärtet sein, beispielsweise mit Härtern des Epoxidtyps, des heterocyclischen Ethylenimins und des Acryloyltyps. Weiterhin ist es auch möglich, die Schichten gemäß dem Verfahren der DE-A-22 18 009 zu härten, um farbfotografische Materialien zu erzielen, die für eine Hochtemperaturverarbeitung geeignet sind. Ferner ist es möglich, die fotografischen Schichten mit Härtern der Diazin-, Triazin- oder 1,2-Dihydrochinolin-Reihe zu härten oder mit Härtern vom Vinylsulfon-Typ. Weitere geeignete Härtungsmittel sind aus den DE-A-24 39 551, DE-A-22 25 230, DE-A-23 17 672 und aus der oben angegebenen Research Disclosure 17 643, Abschnitt XI bekannt.

Weitere geeignete Zusätze werden in der Research Disclosure 17 643 und in "Product Licensing Index" von Dezember 1971, Seiten 107-110, angegeben.

Zur Herstellung farbfotografischer Bilder wird das erfindungsgemäße farbfotografische Aufzeichnungsmaterial mit einer Farbentwicklerverbindung entwickelt. Als Farbentwicklerverbindung lassen sich sämtliche Entwicklerverbindungen verwenden, die die Fähigkeit haben, in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1-(N-ethyl-N-methoxyethyl)-3-methyl-p-phenylendiamin.

Weitere brauchbare Farbentwickler sind beispielsweise beschrieben in J. Amer. Chem. Soc. 73, 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren insbesondere z.B. Ethylendiamintetraessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Beispiel 1

Ein farbfotografisches Aufzeichnungsmaterial für die Colornegativfarbentwicklung wurde hergestellt (Schichtaufbau 1 A), indem auf einen transparenten Schichtträger aus Cellulosetriacetat die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben. Alle Silberhalogenidemulsionen waren pro 100 g $AgNO_3$ mit 0,4 g 4-Hydroxy-6-methyl-1,3,3a,7-tetraazainden stabilisiert.

Schichtaufbau 1 A

| | | |
|---|---|---|
| Schicht 1 | (Antihaloschicht) | |
| | schwarzes kolloidales Silbersol mit | |
| | | 0,2 g Ag |
| | | 1,2 g Gelatine |
| | | 0,1 g UV-Absorber UV-1 |
| | | 0,2 g UV-Absorber UV-2 |
| | | 0,02 g Trikresylphosphat (TKP) |
| | | 0,03 g Dibutylphthalat (DBP) |
| Schicht 2 | (Mikrat-Zwischenschicht) | |
| | Mikrat-Silberbromidiodidemulsion | |
| | (0,5 mol-% Iodid; | |
| | mittlerer Korndurchmesser 0,07 µm) aus | |
| | | 0,25 g $AgNO_3$, mit |

1,0 g Gelatine

Schicht 3 (rotsensibilisierte Schicht, mittelempfindlich)
rotsensibilisierte Silberbromidiodidemulsion
(4,0 mol-% Iodid;
mittlerer Korndurchmesser 0,45 µm)
aus
5,35 g $AgNO_3$, mit
3,75 g Gelatine
0,80 g Cyankuppler C-14
0,53 g Cyankuppler C-17
1,33 g TKP
0,23 g DBP

Schicht 4 (Zwischenschicht)
aus
1,43 g Gelatine
0,74 g Scavenger SC-1

Schicht 5 (grünsensibilisierte Schicht, mittelempfindlich)
grünsensibilisierte Silberbromidiodidemulsion
(4,0 mol-% Iodid;
mittlerer Korndurchmesser 0,45 µm)
aus
3,10 g $AgNO_3$, mit
2,33 g Gelatine
0,775 g Magentakuppler M-12
0,775 g TKP
0,136 g DBP

Schicht 6 (Zwischenschicht)
wie Schicht 4

Schicht 7 (Gelbfilterschicht)
gelbes kolloidales Silbersol
mit
0,09 g Ag,
0,34 g Gelatine

Schicht 8 (blauempfindliche Schicht, mittelempfindlich),
blausensibilisierte Silberbromidiodidemulsion
(4,0 mol-% Iodid;
mittlerer Korndurchmesser 0,45 µm) )
aus
3,46 g $AgNO_3$, mit
1,73 g Gelatine
1,25 g Gelbkuppler Y-20
1,25 g TKP
0,152 g DBP

Schicht 9 (Zwischenschicht)
wie Schicht 4

Schicht 10 (Schutz- und Härtungsschicht)
aus
0,68 g Gelatine
0,73 g Härtungsmittel
(CAS Reg. No. 65411-60-1)

In Beispiel 1 werden außer den bereits erwähnten Kupplern folgende Verbindungen verwendet:

UV-Absorber UV-1

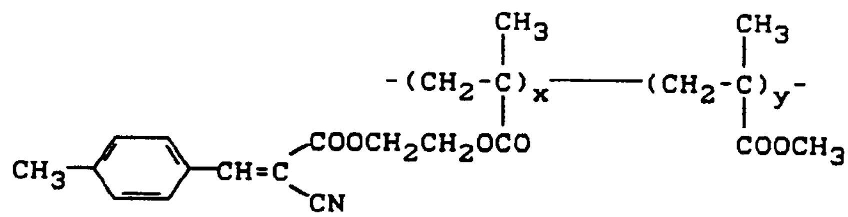

Gewichtverhältnis: x : y = 7:3

UV-Absorber UV-2

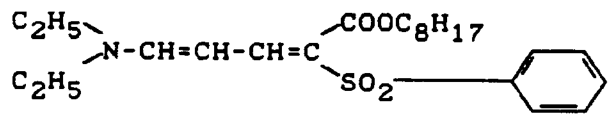

Scavenger SC-1

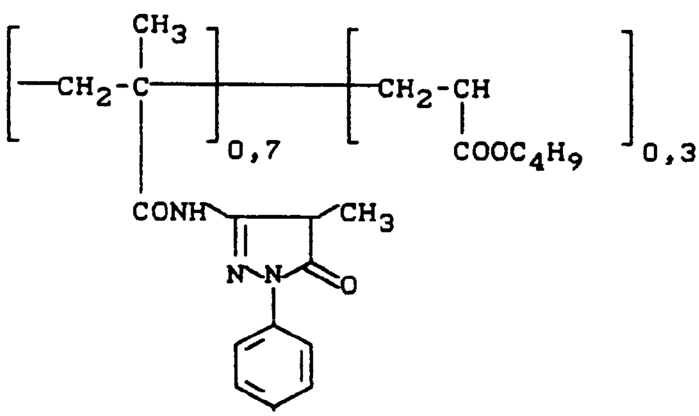

Schichtaufbau 1 A enthielt außerdem in den Schichten 3, 5 und 8 den DIR-Kuppler DIR-1 in einer Menge von 3,1 mmol/100 g $AgNO_3$ (je Schicht).

Weitere Schichtaufbauten 1 B bis 1 L wurden entsprechend hergestellt, die sich von Schichtaufbau 1 A ausschließlich durch den in den Schichten 3, 5 und 8 verwendeten DIR-Kuppler unterscheiden, wie in Tabelle 1 angegeben.

Die Entwicklung wurde nach Aufbelichtung eines Graukeils durchgeführt wie beschrieben in "The British Journal of Photography", 1974, Seiten 597 und 598.

Die Ergebnisse nach Verarbeitung sind in Tabelle 1 dargestellt. Die Interimageeffekte IIE berechnen sich wie folgt:

$$IIE_{bg} = \frac{\gamma_{rot} - \gamma_w}{\gamma_w} ; IIE_{pp} = \frac{\gamma_{grün} - \gamma_w}{\gamma_w} ; IIE_{gb} = \frac{\gamma_{blau} - \gamma_w}{\gamma_w} ;$$

Dabei bedeutet:

| | |
|---|---|
| $\gamma_{rot}$ | Gradation bei selektiver Belichtung mit rotem Licht |
| $\gamma_{grün}$ | Gradation bei selektiver Belichtung mit grünem Licht |
| $\gamma_{blau}$ | Gradation bei selektiver Belichtung mit blauem Licht |
| $\gamma_w$ | Gradation bei Belichtung mit weißem Licht |

Der in Tabelle 1 angegebene Kanteneffekt KE ist die Differenz zwischen Mikro- und Makrodichte bei Makrodichte 0,8, wie beschrieben in James, The Theory of the Photographic Process, 4th Edition, Macmillan Publishing Co., Inc. 1977, Seite 611. Dabei bedeutet:

$KE_{bg}$ KE in der rotsensibilisierten Schicht

$KE_{pp}$ KE in der grünsensibilisierten Schicht

Tabelle 1

| Schicht-aufbau | DIR-Kuppler DIR- | $IIE_{gb}$ | $IIE_{pp}$ | $IIE_{bg}$ | $KE_{pp}$ | $KE_{bg}$ |
|---|---|---|---|---|---|---|
| 1 A | 1 | 48 | 122 | 84 | 0,84 | 1,08 |
| 1 B | 3 | 34 | 102 | 68 | 0,74 | 0,78 |
| 1 C | 4 | 4 | 62 | 38 | 0,41 | 0,63 |
| 1 D | 6 | 24 | 78 | 37 | 0,50 | ››0,50 |
| 1 E | 7 | 5 | 52 | 32 | 0,35 | 0,47 |
| 1 F | 8 | 25 | 95 | 54 | ››0,40 | 0,60 |
| 1 G | 9 | 30 | 106 | 53 | 0,52* | 0,56* |
| 1 H | 12 | 13 | 77 | 42 | 0,27* | 0,36* |
| 1 I | 13 | 9 | 63 | 30 | 0,38 | 0,43 |
| 1 J | 14 | 19 | 105 | 48 | ››0,50 | 0,90 |
| 1 K | 29 | 53 | 134 | 68 | 0,78* | 0,91* |
| 1 L | 30 | 14 | 103 | 46 | 0,68 | 0,70 |

* bei Dichte 0,6

Aus Tabelle 1 geht hervor, daß mit den erfindungsgemäßen DIR-Kupplern ausgezeichnete Interimageeffekte und Kanteneffekte erhalten werden können.

Bei Lagerung der unbelichteten Materialien 1 A bis 1 L (14 Tage bei 60°C und 35 % r.F.) wird nach Belichtung und Verarbeitung nur eine unwesentliche Änderung der sensitometrischen Daten gegenüber den entsprechenden bei Raumtemperatur gelagerten Materialien festgestellt.

## Patentansprüche

1. Farbfotografisches Aufzeichnungsmaterial mit mindestens einer lichtempfindlichen Silberhalogenidemulsionsschicht und einem dieser zugeordneten DIR-Kuppler, dadurch gekennzeichnet, daß der DIR-Kuppler der folgenden allgemeinen Formel I entspricht

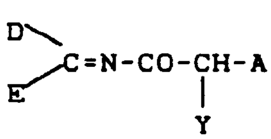

(I)

worin bedeuten

A Halogen, -OR, -SR, -CN, -COR, -CONHR, COOR, $-SO_2R$, - $SO_2NHR$,

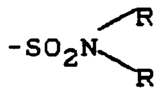 , 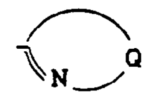 , 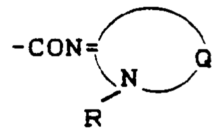

oder 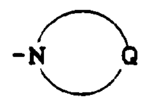 ;

| | |
|---|---|
| R | Alkyl, Aralkyl, Aryl oder Hetaryl; |
| Q | einen Rest zur Vervollständigung eines 5- oder 6-gliedrigen heterocyclischen Ringes; |
| D,E | Reste, die gleich oder verschieden sind und an das C-Atom der C=N-Gruppe über ein C-, O-, S- oder N-Atom gebunden sind, einschließlich solcher Reste, die gemeinsam zusammen mit dem C-Atom der C=N-Gruppe einen carbocyclischen oder heterocyclischen Ring vervollständigen; |
| Y | einen bei Farbentwicklung freisetzbaren Rest mit Silberhalogenidentwicklungsinhibierungsfunktion. |

2. Aufzeichnungsmaterial nach Anspruch 1, dadurch gekennzeichnet, daß der DIR-Kuppler der allgemeinen Formel (II) entspricht

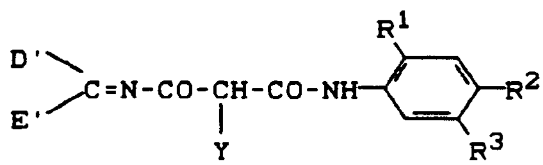 (II)

worin bedeuten

| | |
|---|---|
| D′ und E′ | zusammen einen Rest zur Vervollständigung eines heterocyclischen Ringes, an den ein Benzolring ankondensiert sein kann; |
| $R^1$ | Wasserstoff, Halogen, Alkoxy oder Aroxy; |
| $R^2$ | Wasserstoff, Alkoxy, Alkoxycarbonyl; |
| $R^3$ | Wasserstoff, Sulfamoyl, N-Alkylsulfamoyl, N-Acylsulfamoyl, Alkoxycarbonyl, Acylamino; |
| Y | einen bei Farbkupplung freisetzbaren Rest mit Silberhalogenidentwicklungsinhibierungsfunktion. |

## Claims

1. A color photographic recording material comprising at least one photosensitive silver halide emulsion layer and a DIR coupler associated therewith, characterized in that the coupler corresponds to formula I

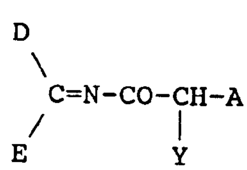 (I)

in which

| | |
|---|---|
| A | represents halogen, -OR, -SR, -CN, -COR, -CONHR, COOR, $-SO_2R$, $-SO_2NHR$, |

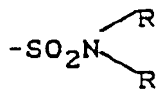 , 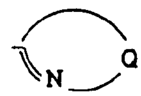 , 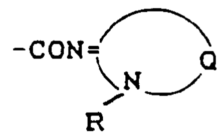

or 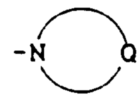 ;

| | |
|---|---|
| R | represents alkyl, aralkyl, aryl or hetaryl; |
| Q | represents a substituent for completing a 5- or 6-membered heterocyclic ring; |
| D and E, | which may be the same or different, represent subtituents attached to the C atom of the C=N group by a C, O, S or N atom, including those substituents which, together with the C atom of the C=N group, complete a carbocyclic or heterocyclic ring; |
| Y | represents a group having a silver halide development inhibiting function which is releasable during color development. |

2. A recording material as claimed in claim 1, characterized in that the DIR coupler corresponds to general formula (II):

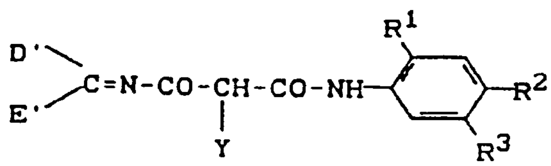 (II)

in which

| | |
|---|---|
| D′ and E′ | together represent a substituent for completing a heterocyclic ring to which a benzene ring may be fused, |
| $R^1$ | is hydrogen, halogen, alkoxy or aroxy; |
| $R^2$ | is hydrogen, alkoxy, alkoxycarbonyl; |
| $R^3$ | is hydrogen, sulfamoyl, N-alkyl sulfamoyl, N-acyl sulfamoyl, alkoxycarbonyl, acylamino; |
| Y | is a substituent having a silver halide development inhibiting function which is releasable during the color coupling reaction. |

## Revendications

1. Matériau d'enregistrement pour photographie en couleurs, comprenant au moins une couche photosensible d'émulsion à l'halogénure d'argent et un copulant DIR attribué à cette dernière, caractérisé en ce que le copulant DIR répond à la formule générale I ci-après :

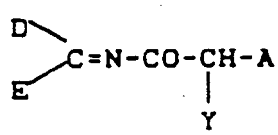 (I)

dans laquelle

A représente un atome d'halogène, -OR, -SR, -CN, -COR, -CONHR, COOR, $-SO_2R$, $-SO_2NHR$;

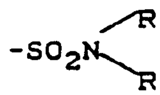 , 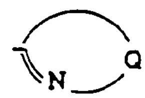 , 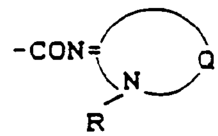

ou 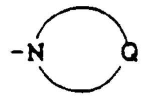 ;

| | |
|---|---|
| R | représente un groupe alkyle, un groupe aralkyle, un groupe aryle ou un groupe hétaryle; |
| Q | représente un radical pour compléter un noyau hétérocyclique penta- ou hexagonal; |
| D,E | représentent des radicaux qui sont identiques ou différents et qui sont liés à l'atome de carbone du groupe C=N via un atome C-, O-, S- ou N-, y compris des radicaux qui complètent, ensemble avec l'atome C du groupe C=N, un noyau carbocyclique ou hétérocyclique; |
| Y | représente un radical séparable lors du développement chromogène, ayant une fonction d'inhibition du développement de l'halogénure d'argent. |

2. Matériau d'enregistrement selon la revendication 1, caractérisé en ce que le copulant DIR répond à la formule générale (II)

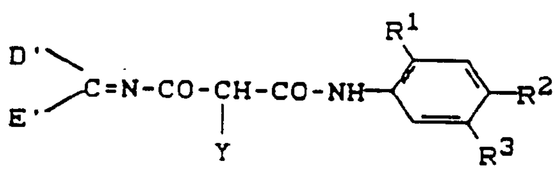 (II)

dans laquelle

| | |
|---|---|
| D' et E' | représentent ensemble un radical pour compléter un noyau hétérocyclique auquel un noyau benzénique peut être condensé; |
| $R^1$ | représente un atome d'hydrogène, un atome d'halogène, un groupe alcoxy ou un groupe aroxy; |
| $R^2$ | représente un atome d'hydrogène, un groupe alcoxy, un groupe alcoxycarbonyle; |
| $R^3$ | représente un atome d'hydrogène, un groupe sulfamoyle, un groupe N-alkylsulfamoyle, un groupe N-acylsulfamoyle, un groupe alcoxycarbonyle, un groupe acylamino; |
| Y | représente un radical séparable lors du développement chromogène, ayant une fonction d'inhibition du développement de l'halogénure d'argent. |